**Europäisches Patentamt**

**European Patent Office**

**Office européen des brevets**

(19)

(11) **EP 0 835 126 B1**

(12) **EUROPEAN PATENT SPECIFICATION**

(45) Date of publication and mention
of the grant of the patent:
**02.04.2003 Bulletin 2003/14**

(51) Int Cl.[7]: **A61K 38/17**, C07K 14/435,
A61P 23/02

(21) Application number: **96923511.8**

(22) Date of filing: **26.06.1996**

(86) International application number:
**PCT/US96/11041**

(87) International publication number:
**WO 97/001351 (16.01.1997 Gazette 1997/04)**

(54) **COMPOSITIONS AND FORMULATIONS FOR PRODUCING ANALGESIA AND FOR INHIBITING PROGRESSION OF NEUROPATHIC PAIN DISORDERS**

ZUSAMMENSETZUNGEN ZUR ERZIELUNG VON ANALGESIE UND ZUR HEMMUNG DER PROGRESSION NEUROPATHISCHER SCHMERZERKRANKUNGEN

COMPOSITIONS ET FORMULATIONS PERMETTANT DE PRODUIRE UNE ANALGESIE ET D'INHIBER LA PROGRESSION DE TROUBLES LIES A DES DOULEURS NEUROPATHIQUES

(84) Designated Contracting States:
**AT BE CH DE DK ES FI FR GB GR IE IT LI LU MC NL PT SE**

(30) Priority: **27.06.1995 US 496847**
**08.03.1996 US 613400**

(43) Date of publication of application:
**15.04.1998 Bulletin 1998/16**

(73) Proprietor: **ELAN PHARMACEUTICALS, INC.**
**Menlo Park, CA 94025-1057 (US)**

(72) Inventors:
• **AMSTUTZ, Gary, Arthur**
**San Jose, CA 95117 (US)**
• **BOWERSOX, Stephen, Scott**
**Menlo Park, CA 94025 (US)**
• **GOHIL, Kishorchandra**
**Richmond, CA 94805 (US)**
• **ADRIAENSSENS, Peter, Isadore**
**Mountain View, CA 94040 (US)**
• **KRISTIPATI, Ramasharma**
**Fremont, CA 94555 (US)**
• **GADBOIS, Theresa**
**Menlo Park, CA 94025 (US)**
• **PETTUS, Mark, Raymond**
**San Jose, CA 95112 (US)**
• **LUTHER, Robert, R.**
**Los Altos Hills, CA 94024 (US)**

(74) Representative: **Barth, Renate, Dr.**
**Vossius & Partner**
**Postfach 86 07 67**
**81634 München (DE)**

(56) References cited:
**WO-A-91/07980** **WO-A-93/10145**
**WO-A-93/13128** **DE-A- 3 926 287**

• **Society for Neuroscience/Abstracts, Volume 20, 1994, A.L. Zhang et al, "Acute Allodynia-Like Responses in Rats after Spinal cord Ischemia: Neuronal Mechanisms and Pharmacological Treatments"**
• **Dialog Information Services, File 351, WPIL Dialog Accession No. 010083868, WPI Accession No. 94-351581/44, Bristol-Myers Squibb Co: "Stabilised injection prepn - comprises deoxythymidine nucleoisede and stabiliser (s) selected from sugar alcohol, benzyl alcohol, phenol, etc"; & JP,A,6247879, 940906, 9444 (Basic)**

## Description

### Field of the Invention

The present invention relates to the use of omega-conopeptides in the manufacture of medicaments for inhibiting the progression of neuropathic pain disorders. The invention also relates to the use of omega conopeptides in the manufacture of medicaments suitable for epidural administration to a patient and to formulations suitable for stabilizing omega conopeptide peptides used in such medicaments.

### Background of the Invention

Analgesic therapies are indicated for treating conditions that have in common the perception of pain by a patient. The most common form of pain, somatic or nociceptive pain, results from any of a variety of noxious or painful stimuli originating in the skin, underlying tissue or viscera. Nociceptive pain results from activation of peripheral nociceptive afferent C- and A-nerve fibers which project to the dorsal horn of the spinal cord and which synapse with afferents to specific brain areas.

Examples of stimuli which evoke nociceptive pain responses are noxious stimuli to the skin (heat, cold), injury of tissues such as lacerations to the skin and underlying tissues, and inflammation. Relief from nociceptive pain may be provided by non-narcotic analgesics, such as aspirin, ibuprofen or indomethacin, or by opioid compounds, which have a direct inhibitory action on the firing of C-fibers. Certain local anesthetics (*e.g.*, bupivicaine), antihistamines, amphetamines and antidepressants may be used to enhance the effects of opioids. In addition, co-owned U.S. Patent 5,364,842 discloses the effectiveness of N-type calcium channel blocking omega-conopeptide compositions in producing analgesia or augmenting opioid analgesia.

Neuropathic pain is pain that results from injury to, or chronic changes in, peripheral or central somatosensory pathways, collectively referred to herein as "neuropathic pain disorders." It is generally a chronic condition that has a complex and variable etiology. The underlying peripheral nerve damage which causes neuropathic pain is typically attributable to trauma or injury to the peripheral nerve, nerve plexus or to the soft tissue surrounding the nerve. Neuropathic pain can also be a consequence of one or more of a number of underlying pathologies, including cancer, bone degenerative diseases, and AIDS.

Neuropathic pain presents in various forms, having both provoked and unprovoked (spontaneous) components. The provoked component of neuropathic pain usually involves hypersensitivity to sensory stimuli and may include hyperalgesia (exaggerated response to a noxious stimulus), hyperesthesia (exaggerated response to touch) and allodynia (pain perception from innocuous mechanical or thermal stimuli). Patients may also experience spontaneous pain which has a variety of manifestations. One manifestation, termed "causalgia" is typical of nerve injury and is characterized by continuous, severe burning pain and allodynia. Neuralgia is a form of neuropathic pain that results from trauma or irritation to a peripheral nerve and has symptoms consistent with its focal etiology. In some cases (such as trigeminal neuralgia), the patient experiences paroxysmal, lancinating pain. Diffuse sensory neuropathies, in contrast, present with symmetric, distal pain that typically affects the feet and hands. Neuropathic pain also results from damage to central somatosensory pathways, such as the ascending somatosensory pathway at the level of the spinal cord, brainstem, thalamus or cortex. Such lesions may result in a syndrome of continuous, spontaneous pain that is felt in the periphery.

Currently, clinicians may attempt to manage neuropathic pain with the same drugs that are effective in treating nociceptive pain; however, results are generally disappointing. In addition to non-narcotic analgesics, opioids and local anesthetics, attempts to treat neuropathic pain have included the use of anticonvulsants (phenytoin), antisympathetics, tricyclic antidepressants and transcutaneous nerve stimulation. WO 93/13128 discloses that N-type calcium channel blocking omega conopeptides produce relief from neuropathic pain.

A complication of a neuropathic pain disorder is its progressive nature which results in a worsening of symptoms of neuropathic pain over time. The etiology of this degeneration is not well understood; however, it appears that damaged nerves and adjacent nerves become more sensitive to low levels of stimulation.

The present invention is based on the discovery that N-type voltage-sensitive calcium channel (VSCC) blocking omega-conopeptides prevent progression of neuropathic pain disorders, particularly when such compounds are delivered to the site of the neuropathic pain such that pain symptoms do not worsen with time.

The present invention also discloses a novel route of administration of omega conopeptides for pain management. In view of the hydrophilic, charged nature of omega conopeptides and the permeation properties of the spinal meninges, it was previously believed that epidural administration of omega-conopeptides would require addition of a membrane permeation enhancer. According to one discovery of the present invention, omega conopeptides can provide pain relief when administered epidurally in the absence of a membrane permeation enhancer, at doses that are comparable to effective analgesic doses using intrathecal administration (*e.g.*, direct delivery to the spinal fluid). As described herein, such epidural administration is particularly effective when it is carried out in such a way that the administered compound is placed in prolonged contact with the epidural space, and more particularly, with the spinal meninges. The improved method has the advantage that epidural administration is technically less demanding than intraspinal administration of compound and poses fewer risks to the patient.

The present invention also discloses improved formulations for omega conopeptides that confer stability to solutions containing the compounds. Such formulations are particularly useful in the prolonged (continuous administration) treatment methods. These stabilized formulations are also useful for long-term storage of conopeptide solutions.

### Summary of the Invention

In one aspect, the invention includes the use of an N-type calcium channel antagonist omega conopeptide in the manufacture of a medicament for inhibiting progression of a neuropathic pain disorder characterized by neuropathic pain, when the compound is administered to a subject at the site of progression of nerve injury. The omega conopeptide is characterized by its ability to (a) inhibit electrically stimulated contraction of the guinea pig ileum, and (b) bind selectively to an omega conopeptide MVIIA binding site present in neuronal tissue, as evidenced by the ability of the compounds to displace MVIIA from the site.

According to preferred embodiments, the omega conopeptide used in the medicament is further characterized by:

(i) activities in such guinea pig ileum contraction inhibition and in MVIIA binding site selective binding that are within the ranges of such activities of omega-conotoxins MVIIA and TVIA;
(ii) activity in MVIIA site selective binding that is evidenced by a selectivity ratio of binding affinity for the MVIIA binding site to binding affinity for a site 2 omega conopeptide binding site which is within the range of selectivity ratios determined for omega conopeptides MVIIA/SNX-111, SNX-199, SNX-236, SNX-239 and TVIA/SNX-185;
(iii) inclusion of an anti-oxidant effective to prevent methionine oxidation;
(iv) use by perineural administration in inhibiting progression of a neuropathic pain disorder at an injured nerve site; or
(v) selection from the omega conopeptides TVIA/SNX-185, MVIIA/SNX-111, SNX-236, SNX-159, SNX-239, SNX-199, SNX-273, SNX-279, and derivatives thereof.

In another aspect, the invention includes a stable omega conopeptide formulation which includes an omega conopeptide and a carboxylic acid buffer anti-oxidant composition. In a preferred embodiment, the carboxylic acid buffer is lactate buffer and may also include free methionine.

In another aspect, the invention includes the use of an N-type voltage-sensitive calcium channel blocking omega conopeptide in the manufacture of a medicament for producing analgesia in a subject by epidural administration. The omega conopeptide used in this medicament has the same *in vitro* activities as described above. The epidural medicament is provided at a dosage that is in the range of an effective intrathecal analgesic dose administered over an equivalent period of time. In preferred embodiments, the omega-conopeptide selected for use in the medicament is characterized by:

(i) a selectivity ratio of binding to an MVIIA binding site and to binding to a site 2 omega conopeptide binding site which is within the range of selectivity ratios determined for omega conopeptides MVIIA/SNX-111, SNX-199, SNX-236, SNX-239 and TVIA/SNX-185;
(ii) inclusion in the medicament of an omega conopeptide selected from the group consisting of MVIIA/SNX-111, TVIA/SNX-185, SNX-236, SNX-159, SNX-239, SNX-199, SNX-273, SNX-279, and derivatives thereof;
(iii) administration of the medicament by continuous infusion; or
(iv) inclusion of a sustained release formulation to effect prolonged contact of the conopeptide with the epidural region.

In other preferred embodiments, the medicament for epidural administration may include one or more of omega-conopeptides MVIIA/SNX-111, TVIA/SNX-185, SNX-236, SNX-159, SNX-239, SNX-199, SNX-273, SNX-279, and derivatives thereof.

In still other, related embodiments, the invention includes omega-conopeptide SNX-273 having the sequence: CKGKGAKCSRLAYDCCTGSCRSGKC. The invention also includes an omega-conopeptide SNX-279 having the sequence CKGKGAKCSRLM(0)YDCCTGSCRSGKC, where M(O) indicates an oxidized methionine residue. Both these omega-conopeptides have analgesic properties and are suitable for inclusion in the medicaments described above.

These and other objects and features of the invention will become more fully apparent when the following detailed description of the invention is read in conjunction with the accompanying drawings.

**Brief Description of the Drawings**

Figures 1 and 2 show primary sequences of several natural omega-conopeptides, MVIIA/SNX-111 (SEQ ID NO: 01), MVIIB/SNX-159 (SEQ ID NO: 02), GVIA/SNX-124 (SEQ ID NO: 03), GVIIA/SNX-178 (SEQ ID NO: 04), RVIA/SNX-182 (SEQ ID NO: 05), SVIA/SNX-157 (SEQ ID NO: 06), TVIA/SNX-185 (SEQ ID NO: 07), SVIB/SNX-183 (SEQ ID NO: 08), and MVIIC/SNX-230 (SEQ ID NO: 29), and SNX-231 (SEQ ID NO: 21);

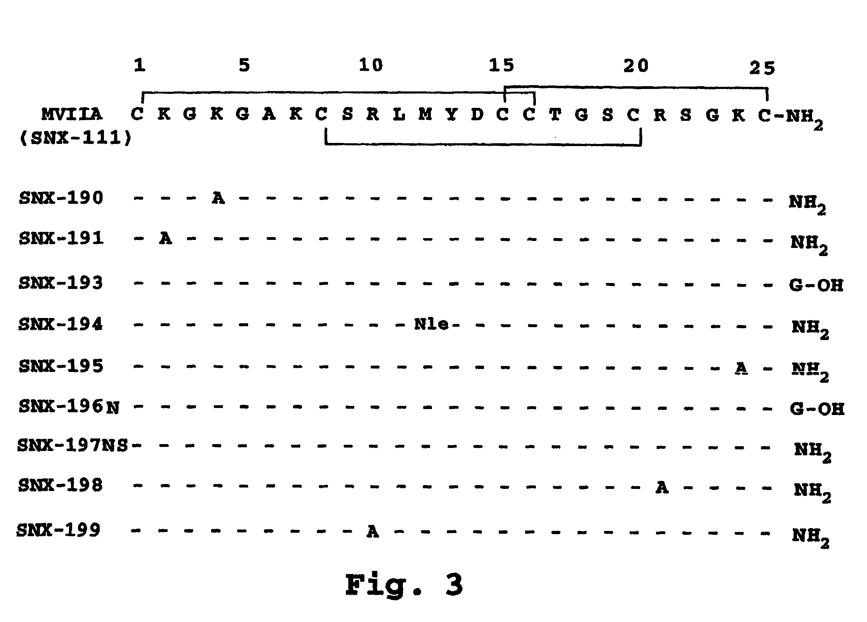

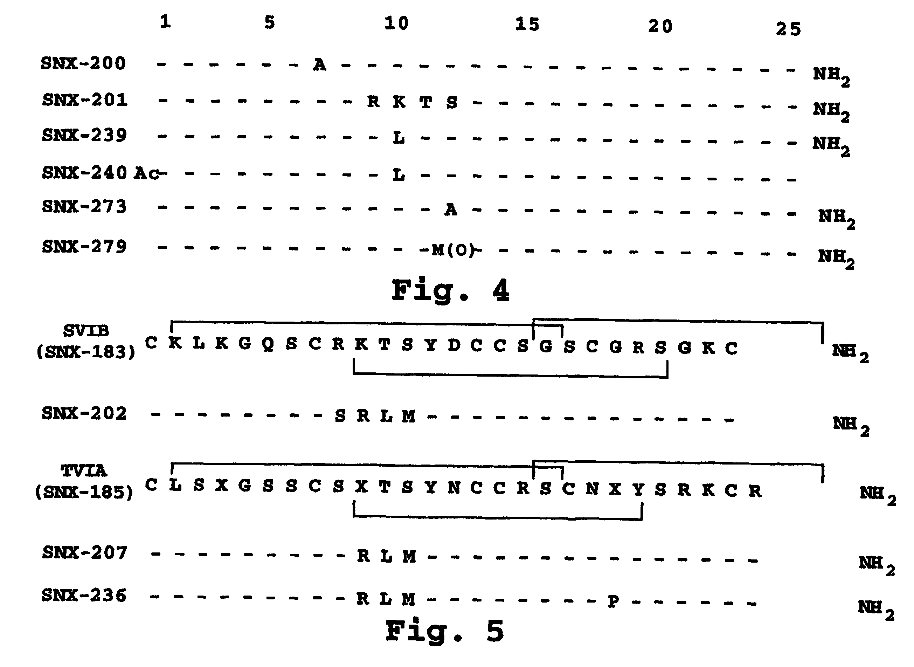

Figures 3 and 4 show primary sequences of MVIIAISNX-111, including its disulfide bonding pattern and SNX-111 analog omega-conopeptides SNX-190 (SEQ ID NO: 09), SNX-191 (SEQ ID NO: 10), SNX-193 (SEQ ID NO: 11), SNX-194 (SEQ ID NO: 12), SNX-195 (SEQ ID NO: 13), SNX-196 (SEQ ID NO: 14), SNX-197 (SEQ ID NO: 15), SNX-198 (SEQ ID NO: 16), SNX-199 (SEQ ID NO: 33), SNX-200 (SEQ ID NO: 17), SNX-201 (SEQ ID NO: 18), SNX-239 (SEQ ID NO: 32), SNX-240 (SEQ ID NO: 34), SNX-273 ([ala$^{12}$-SNX-111; SEQ ID NO: 35), and SNX-279 (Met(0)$^{12}$-SNX-111; SEQ ID NO: 36), where Nle indicates norleucine, and Met(0) indicates a sulfoxy-methionine substitution;

Figure 5 shows SVIB/SNX-183 analog SNX-202 and TVIA/SNX-185 analogs SNX-207 and SNX-236;

Figure 6 shows omega-conopeptide groupings: I, MVIIA, SNX-199 (SEQ ID NO: 33), MVIIB and SNX-239 (SEQ ID NO: 32), II, TVIA, SNX-207 and SNX-236, and III, RVIA, SVIA, GVIIA, SVIB, MVIIC, SNX-231;

Figure 7 shows voltage-gated calcium current traces induced by a voltage step in untreated N1E-115 neuroblastoma cells (curve a) and in neuroblastoma cells exposed to increasing concentrations of MVIIA (SNX-111) (curves b-d);

Figure 8 shows computer-fit competitive binding curves for omega-conopeptide binding to the MVIIA (SNX-111) binding site in rat brain synaptosomes;

Figures 9 shows computer-fit competitive binding curves for omega-conopeptide binding to the MVIIC (SNX-230) binding site in rat brain synaptosomes;

Figure 10 shows analgesic efficacy of an SNX-111 formulation combining methionine in lactate buffer;

Figure 11 shows a plot of displacement of [$^{125}$I]SNX-111 binding to monkey pia arachnoid matter homogenates by [Nle$^{12}$]SNX-111;

Figure 12 shows histograms of thresholds of mechanical allodynia as %MPE at various times after bolus epidural injection of 30 μg SNX-111;

Figure 13 shows histograms of thresholds of mechanical allodynia as %MPE after 24, 72, 120 or 168 hours of continuous intrathecal infusion of saline or 0.1μg/hour SNX-111; and

Figure 14 shows histograms of thresholds of mechanical allodynia as %MPE after 24, 48 or 72 hours of constant epidural infusion of saline or varying amounts of SNX-111 into the spinal lumbar epidural space of rats.

Detailed Description of the Invention

I. Definitions:

Unless indicated otherwise, the terms below have the following definitions:

"Pain" includes both somatic (nociceptive) and neuropathic pain.

"Somatic pain" and "nociceptive pain" are used interchangeably to refer to pain that results from any of a variety of noxious or painful stimuli originating in the skin, underlying tissue or viscera and which activate peripheral nociceptive afferent C- and A-nerve fibers which project to the dorsal root of the spinal cord and which synapse with afferents to specific brain areas.

"Neuropathic pain" results from injury to, or chronic changes in, peripheral or central somatosensory pathways, complex and variable etiology.

The underlying peripheral nerve damage which causes neuropathic pain is typically attributable to trauma or injury to a peripheral nerve, nerve plexus or soft tissue surrounding the nerve. Neuropathic pain can also be a consequence of one or more of a number of underlying pathologies, including cancer, bone degenerative diseases, and AIDS. This type of pain may present in a number of forms, as discussed above; however, it typically includes hypersensitivity to sensory stimuli which may be accompanied by a persistent sensation of pain in the afflicted region. These varied underlying causes of neuropathic pain are collectively termed "neuropathic pain disorders."

"Progression of a neuropathic pain disorder" refers to the worsening of the symptoms of neuropathic pain over time. A therapeutic regimen is said to inhibit or retard progression of a neuropathic pain disorder, if it prevents or inhibits worsening of such symptoms. Specific examples of neuropathic pain disorders include, but are not limited to, central neuropathies, such as may be caused by insult to the spinal cord, peripheral neuropathies such as reflex sympathetic dystrophy (RSD), herpes zoster neuropathy, and diabetic neuropathy, neuropathies that are a consequence of insult to peripheral nerves, as well as neuropathic pain associated with unknown causes but presenting with the classic symptoms of neuropathic pain, such as hyperesthesia, allodynia, and hyperalgesia.

"Perineural administration" means application of therapeutic agent to or near a nerve, particularly a damaged nerve.

"Epidural administration" refers to administration of a compound to the epidural space, defined as the region between the dura mater and arachnoid membrane surrounding the spinal cord within the spinal canal.

"Intrathecal administration" refers to administration of a compound into the subarachnoid space of the spinal cord; by subarachnoid space is meant the region between the arachnoid membrane and the pia mater surrounding the spinal cord.

Administration of a medicament "at the site of progression of nerve injury" includes any means that will place the medicament in the close proximity of an affected nerve, ganglion, spinal synapses, or affected tissue region. Modes of administration included by this term include, but are not limited to, perineural, epidural, intrathecal, and intravenous administration.

"Active omega conopeptides" refers to peptides that (a) block N-type voltage-sensitive calcium channels, (b) conform to certain structural constraints, and (c) have specified *in vitro* activities in (i) inhibiting electrically-stimulated contractions of guinea pig ileum, and (ii) binding to MVIIA binding sites in neuronal membranes. The peptides are 25-35 amino acids in length and conform to other sequence constraints as described in Section IIA, herein. The *in vitro* activities are preferably within the range of activities measured for omega conopeptides MVIIA and TVIA in the appropriate assays.

II. Preparation of Therapeutic Medicament

This section describes the preparation of a an omega conopeptide composition for use in inhibiting the progression of a neuropathic pain disorder, in accordance with the invention.

A. Active Omega-Conopeptides

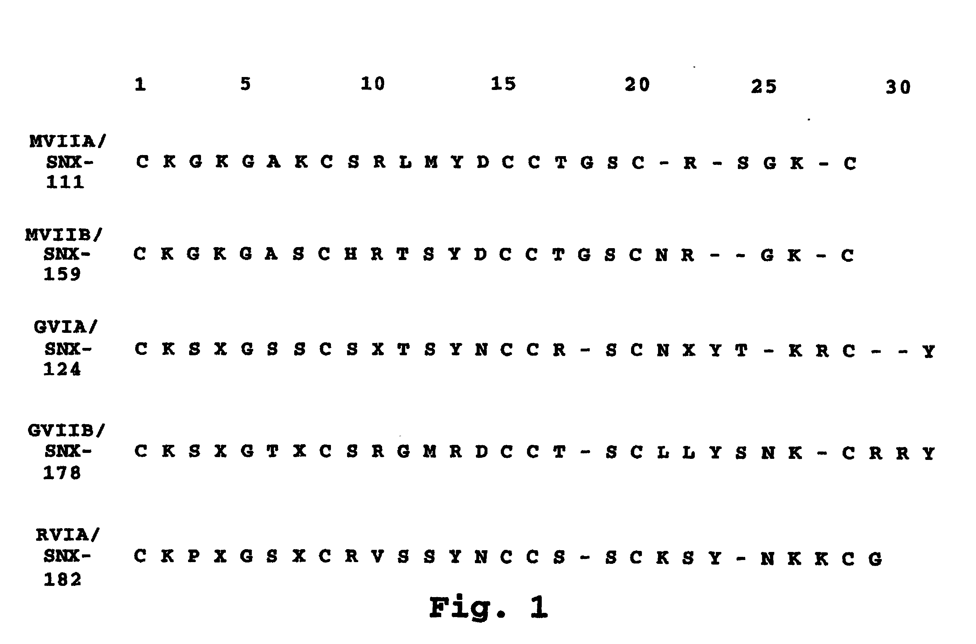

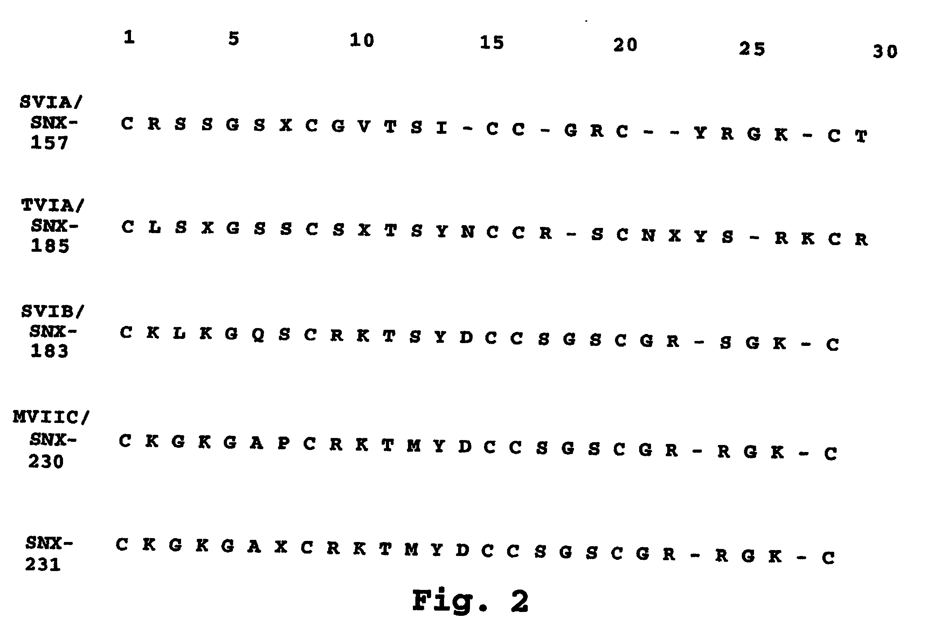

Omega-conopeptides are components of or derived from peptide toxins produced by marine snails of the genus *Conus,* which act as calcium channel blockers (Gray, *et al.*, 1988). The primary sequences of eight naturally-occurring omega-conopeptides are shown in Figures 1 and 2, where SNX-231 is an alternative form of MVIICISNX-230. Conventional letter initials are used for the amino acid residues, and X represents 4-hydroxyproline, also abbreviated 4Hyp. All of the peptides shown in the figure are amidated at their C-termini.

1. Structural Characteristics of Active Omega Conopentides

Omega-conopeptides useful in forming the medicaments of the present invention conform to certain physical and chemical constraints, as described below. Generally, omega-conopeptides useful in the treatment method are 25-35 amino acids in length and have three disulfide bonds at specified positions. This section provides further guidance for selection of analgesic omega-conopeptides; however, it is appreciated that the guidelines provided below are subservient to the *in vitro* predictive assays described herein.

Based on a sequence homology analysis of the peptides whose full sequences are known (Figure 6), the naturally occurring active omega-conopeptides may be grouped into distinct groups I and II, each with internal homologies distinct to that group, as can be appreciated from Figure 6. A third group includes inactive peptides SNX-231, and SVIA (SNX-157) and omega-conopeptides whose binding activities for the MVIIA site on neuronal membranes and/or activity in norepinephrine inhibition are outside the range of active compounds.

The three groups of omega-conopeptides are arranged in Figure 6 with their six Cys residues aligned, which places these residues at positions 1, 8, 15, 16, 20, and 28. To make this alignment, gaps were introduced at the positions shown in the three groups. In the analysis below, these gaps retain the assigned number shown in the figure, even though they represent amino acid deletions in the respective groups of active omega-conopeptides.

Sequence variation in the peptides, based on primary structure alone, was analyzed by adopting the following constraints:

1. The peptides in both active groups (I and II) include the Cys residues at position 1, 8, 15, 16, 20, and 28.

2. The peptides in the active groups include three disulfide linkages connecting the Cys residues at positions 1 and 16, 8 and 20, and 15 and 28. This constraint excludes amino acid variations in other positions which prevent or otherwise hinder the formation of the three selected bridges.

3. Within Group I, the amino acid variations which occur at the seven non-conserved residues are allowed, including peptides in which the carboxy terminus is amidated or has a free acid form. That is, the first group compound derivatives include the peptide structures having the form: C K G K G A-$X_1$-C-$X_2$-$X_3$-$X_4$-$X_5$-Y D C C T G S C-$X_6$-R-$X_7$-G K C-t, where $X_1$=K or S; $X_2$=S or H; $X_3$=R, L, or A; $X_4$=L or T; $X_5$=M or S; $X_6$= N or a deletion; $X_7$=S or deletion, and t= a carboxy or amidated carboxyterminal group.

4. Within Group II, the amino acid variations which occur at the four non-conserved residues are allowed, including peptides in which the carboxy terminus is amidated or has a free acid form. Thus, the second group compound derivatives include the peptide structures having the form: C L S X G S S C S-$X_1X_2X_3$-Y N C C R S C N-$X_4$-Y S R K C R-t, where $X_1$=X or R; $X_2$=T or L; $X_3$= S or M, $X_4$= X or P; and t= a carboxy or amidated carboxyterminal group.

5. Considering both active groups together, amino acid positions which are conserved in all active species are preserved. Thus, for example, the Cys residues, the 5-position glycine, the 13-position tyrosine, the 19-position serine, and the 26-position lysine are all preserved. Preferred OCT analogs or derivatives may be selected by comparing, for purposes of inter-sequence conservation and substitution, those sequences known to be active.

6. Considering both active groups together, there are amino acid positions which are likely to be variable within the range of active species. For example, the position 2 amino acid may be lysine or leucine, the position-3 amino acid may be glycine or serine, and the position 4 amino acid, hydroxyproline or arginine. In addition, if the two or more amino acids at a variant position are in a common substitution class, substitution within that class may be favorable. Standard substitution classes are the six classes based on common side chain properties and highest frequency of substitution in homologous proteins in nature, as determined, for example, by a standard Dayhoff frequency exchange matrix (Dayhoff). These classes are Class I: Cys; Class II: Ser, Thr, Pro, Hyp, Ala, and Gly, representing small aliphatic side chains and OH-group side chains; Class III: Asn, Asp, Glu, and Gin, representing neutral and negatively charged side chains capable of forming hydrogen bonds; Class IV: His, Arg, and Lys, representing basic polar side chains; Class V: Ile, Val, and Leu, representing branched aliphatic side chains, and Met; and Class VI: Phe, Tyr, and Trp, representing aromatic side chains. In addition, each group may include related amino acid analogs, such as ornithine, homoarginine, N-methyl lysine, dimethyl lysine, or trimethyl-lysine in class IV, and a halogenated tyrosine in Group VI. Further, the classes may include both L and D stereoisomers, although L-amino acids are preferred for substitutions.

7. Considering the known inactive species (Group III), substitutions to amino acids which are present in inactive species, but not active ones, at any selected residue position, are not favored to preserve activity in the active compounds.

The above amino acid selection rules 6-7 are intended as a guide for allowed amino acid substitutions within active omega-conopeptides. Once an amino acid substitution or modification is made, the peptide is further screened for the requisite *in vitro* properties that are identified by the present invention as predictive of calcium channel antagonist activity, and the requisite *in vivo* activities in appropriate animal models of pain.

2. *In vitro* and Pharmacological Properties of Active Omega Conopeptides

This section describes the *in vitro* properties of active omega conopeptides. Active omega conopeptides (i) block N-type voltage-sensitive calcium channels, (ii) are selective, specific binding to the omega conopeptide MVIIA (site 1) binding site in neuronal membranes, and (iii) inhibit neurotransmitter release in the guinea pig ileum contraction assay, as discussed below.

a. Calcium-Channel Antagonist Activity.

Active omega conopeptides inhibit transmission of calcium ions through N-type voltage-sensitive calcium channels (VSCC). Blockade of N-type VSCC can be measured in an isolated cell system, such as the mouse neuroblastoma cell line, strain N1E115 or the human neuroblastoma cell line IMR32, as described in U.S Patent 5,364,842. N-type calcium currents are blocked by omega conopeptide MVIIA, but not by dihydropyridines. Membrane currents are conveniently measured with the whole cell configuration of the patch clamp method, according to the procedure detailed in Example 1.

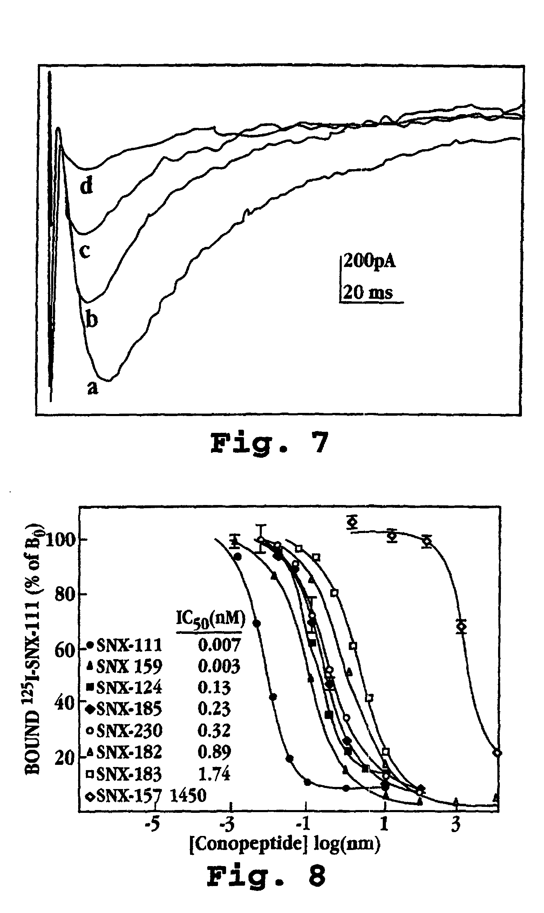

Figure 7 shows a trace of an inward calcium current elicited by a voltage step from -80 mV to -20 mV, where barium (Ba) replaced calcium (Ca) as the charge-carrier through the calcium channels (McCleskey, E.W., *et al., Proc. Natl. Acad. Sci. USA* 84:4327-31 (1987)). Curve a shows a control trace, in the presence of nifedipine, an L-type calcium channel blocker which had no effect on the current characteristics of the channel. Curves *b-d* show blockade of the channel by addition of increasing concentrations of omega conopeptide MVIIA. Conopeptides used in the medicaments of the present invention also block this current.

b. <u>Specific, High Affinity Binding to Omega-Conopeptide Receptors.</u>

A characteristic of active omega-conopeptides is their ability to bind to a specific population of binding sites present mainly in neuronal tissue. Dihydropyridines and other L-type channel blockers do not displace omega conotoxin binding from these sites.

This binding affinity can be characterized either by the binding constant of the compound for the high-affinity MVIIA (SNX-111) binding site, also referred to as "site 1" herein, and/or the binding constant of the compound for the high-affinity SVIB (SNX-183) or the MVIIC (SNX-230) binding site, also referred to as "site 2" herein. Characteristics of these two distinct binding sites are summarized below. It is also useful to characterize omega-conopeptides according to the ratio of their binding constants measured for binding to the two binding sites.

Binding to the MVIIA binding site in neuronal tissue can be demonstrated in a variety of cell types and synaptosomal cell fractions. One preferred synaptosomal fraction is a mammalian brain synaptosomal membrane preparation, such as the rat brain synaptosome preparation described in Example 2 herein. The binding constant of a compound for the MVIIA binding site is typically measured in a competitive displacement assay, where a test compound competes with radiolabeled MVIIA (SNX-111) for binding to the synaptosomal preparation.

Using conventional Scatchard analysis of an equilibrium saturation binding experiment, a $K_d$ binding constant of approximately 10 pM was calculated for binding of omega conopeptide MVIIA to rat synaptosomal membranes. Similarly $K_d$'s were determined for binding of radiolabelled SVIB (SNX-183) and MVIIC (SNX-230) to binding sites in synaptosomal membranes.

To determine the binding constant of a test N-type VSCC blocking compound for a binding site, the test compound is added, at increasing concentrations, to a membrane preparation, such as a synaptosome preparation, in the presence of a standard concentration of radiolabeled omega-conopeptide MVIIA (SNX-111). The synaptosomal material is then rapidly filtered, washed and assayed for bound radiolabel. Competitive displacement binding curves, such as shown in Figure 8 for displacement of MVIIA binding, are analyzed to determine first the $IC_{50}$ value of the compound for the MVIIA binding site, *i.e.*, the concentration which gives 50% displacement of labeled MVIIA peptide. A $K_i$ is determined according to standard methods from the $K_d$ value of MVIIA and the $IC_{50}$ value of the compound, as detailed in Example 2. A relative potency value can also be calculated from this information, as described. Like the $K_i$ value, this value allows comparisons between assays performed under slightly differing conditions or at different times. While the specific $IC_{50}$ value for a particular compound may vary from preparation to preparation, the rank order of binding affinities among the compounds should remain essentially unchanged between experiments. When testing a compound to determine whether it will be useful for inclusion in a medicament of the present invention, the potency of the test compound is compared to standard compounds (such as MVIIA and GVIA) within a given preparation, to determine whether the test compound displaces MVIIA binding within a potency range considered useful in the methods of the invention, as discussed below.

Calculated $IC_{50}$ values for a number of omega-conopeptides for binding of MVIIA (SNX-111) to an MVIIA ("Site I") binding site in a rat synaptosomal preparation are given in Table 1. The compounds are arranged in order of increasing $IC_{50}$ values, where lower values are associated with higher affinity for the binding site.

Table I

| COMPETITION OF $^{125}$I-MVIIA (SNX-111) BINDING BY OMEGA-CONOPEPTIDES | |
| --- | --- |
| | $IC_{50}$ (nM) |
| SNX-207 | .007 |
| SNX-194 | .008 |
| SNX-195 | .009 |
| MVIIA (SNX-111) | .010 |
| SNX-190 | .021 |
| SNX-236 | .030 |
| SNX-200 | .039 |
| SNX-201 | .046 |
| SNX-202 | .046 |
| SNX-193 | .070 |
| SNX-194 | .090 |

Table I   (continued)

| COMPETITION OF $^{125}$I-MVIIA (SNX-111) BINDING BY OMEGA-CONOPEPTIDES | |
| --- | --- |
| | $IC_{50}$ (nM) |
| SNX-239 | .090 |
| MVIIC (SNX-230) | .32 |
| MVIIB (SNX-159) | .101 |
| GVIA (SNX-124) | .134 |
| SNX-198 | .160 |
| SNX-191 | .165 |
| TVIA (SNX-185) | .228 |
| SNX-196 | .426 |
| RVIA (SNX-182) | .893 |
| SVIB (SNX-183) | 1.5 |
| GVIIA (SNX-178) | 3.70 |
| SNX-197 | 11.3 |
| SVIA (SNX-157) | 1460. |

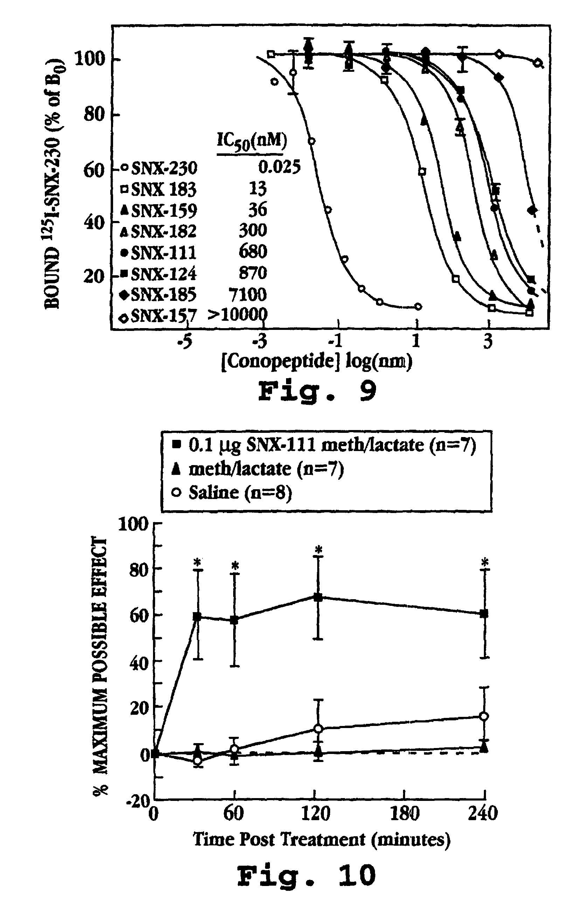

Similarly, $IC_{50}$ and $K_i$ values for compound binding to the "Site 2" (SVIB/SNX-183 or MVIIC/SNX-230) binding site can be calculated, as above, by determining the $K_d$ of labeled SVIB (SNX-183) or MVIIC (SNX-230) binding to a synaptosome preparation, then using competitive displacement of the labeled compound by the test compound, to determine the $IC_{50}$ and $K_i$ or relative potency values of the test compound. Figure 9 shows computer-fit competitive binding curves for several omega-conopeptides whose binding to the SVIB (SNX-183) and/or MVIIC (SNX-230) binding sites was examined. From these curves, $IC_{50}$ values were determined.

Tables 2 and 3 lists the relative potencies for binding of various omega-conopeptides to the site 1 and site 2 binding sites. These data illustrate how either $K_i$ (Table 2) or $IC_{50}$ (Table 3) values can be used to calculate selectivity ratios for binding to the sites.

Generally, omega conopeptides useful in forming the medicaments of the present invention will have binding affinities for the MVIIA site or selectivity ratio for the MVIIA site with reference to site 2, within the range of affinities determined for omega conopeptides MVIIA and TVIA. More generally, the range of selectivity ratios will be defined by omega conopeptides MVIIA/SNX-111, SNX-199, SNX-236, SNX-239 AND TVIA/SNX-185.

## Table 2

### SELECTIVITY OF CONOPEPTIDES FOR SITE 1 AND SITE 2

| Compound | Ki (nM) for competition[a] with: | | Selectivity[b] for: | |
|---|---|---|---|---|
| | [125I]-SNX-111 | [125I]-SNX-230 | site 1 | site 2 |
| SNX-111 | 0.002 | 150 | 75,000 : | 1 |
| SNX-183 | 0.43 | 6 | 14 : | 1 |
| SNX-230 | 0.20 | 0.03 | 1 : | 7 |

[a]Ki values were derived from analysis of competitive binding performed as described in Figure 8.

[b]Selectivity is expressed as the ratio of the Ki value determined for competition with high-affinity [125I]-SNX-230 binding divided by the Ki value for competition with [125I]-SNX-111 binding.

## Table 3

### SELECTIVITY OF CONOPEPTIDES FOR SITE 1 AND SITE 2

| Compound | IC50 (nM) for competition with: | | Selectivity[a] for: | |
|---|---|---|---|---|
| | [125I]-SNX-111 | [125I]-SNX-230 | site 1 | site 2 |
| SNX-199 | 0.09 | 5,000 | 56,000 : | 1 |
| SNX-236 | 0.03 | 1,500 | 50,000 : | 1 |
| SNX-239 | 0.09 | 10,000 | 111,000 : | 1 |

[a]Selectivity is expressed as the ratio of the IC50 value determined for competition with [125I]-SNX-230 binding divided by the IC50 value for competition with [125I]-SNX-111 binding.

c. Selective Inhibition of Neurotransmitter Release

Active omega-conopeptides also inhibit neurotransmitter release in various regions of the nervous system. Neurotransmitters which can be measured, in accordance with various aspects of the invention, include, but are not limited to dopamine, norepinephrine, acetylcholine, GABA, glutamate, and a number of peptide neurotransmitters, such as calcitonin gene-related peptide.

Isolated tissue assays are traditional pharmacological methods for measuring effects on neurotransmitter release. One such preparation is the guinea pig ileum, where inhibition of electrically stimulated contractions correlates with inhibition of acetylcholine release. Example 4 describes the preparation and assay in detail. Table 4 shows the $IC_{50}$ values for various omega-conopeptides on contraction of guinea pig ileum in response to electrical stimulation. Active omega conopeptides SNX-111 and SNX-185 are most potent in inhibiting such contraction, while inactive compounds SNX-183 and SNX-157 are much less potent.

Table 4

| EFFECTS OF CONOPEPTIDES ON ELECTRICALLY STIMULATED CONTRACTION OF GUINEA PIG ILEUM | |
| --- | --- |
| Compound | $IC_{50}$ (nM) |
| SNX-111 | 13 |
| SNX-185 | 29 |
| SNX-183 | 91 |
| SNX-157 | >100 |

In summary, N-type VSCC blocking omega conopeptides that are useful in the manufacture of medicaments of the present invention exhibit certain *in vitro* and pharmacological properties in inhibiting electrically stimulated contraction in the guinea pig ileum and in binding selectively to MVIIA binding sites in neuronal tissue, as observed for omega conopeptides MVIIA/SNX-111, TVIAISNX-185, SNX-236, SNX-159, SNX-239, SNX-199, SNX-273, and SNX-279.

Once selected for use in a medicament according to the foregoing *in vitro* criteria, the active omega-conopeptide can be tested in any of a number of experimental animal models of neuropathy to determine dosage ranges for humans.

B. Preparation of Omega-Conopeptides

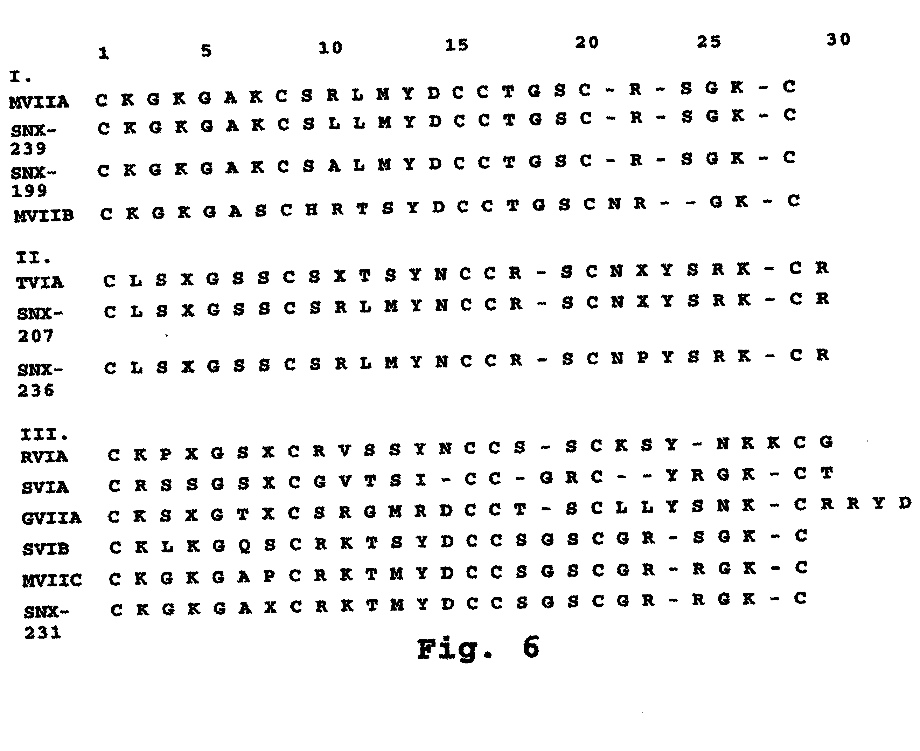

Omega-conopeptides can be produced by conventional synthetic methods or isolated from natural sources. Synthetic and naturally occurring peptides having the same sequence behave substantially identically when used in the medicaments described herein. All of the omega-conopeptides shown have three disulfide linkages connecting cysteine residues 1 and 4, 2 and 5, and 3 and 6, as indicated for the MVIIA peptide in Figure 3. Figures 3-5 show analogs or derivatives of natural omega conopeptides MVIIA, TVIA, and SVIB which have been synthesized and tested in accordance with the invention. Standard single amino acid code letters are used in the Figures; Additionally, the following abbreviations are used: X=hydroxyproline; Nle=norleucine; Met(O)=sulfoxy-methionine; $NH_2$ group at the C terminus indicates that the peptide is C-terminal amidated; G-OH indicates termination in an unmodified glycine residue.

Many omega conopeptides are available from commercial sources (*e.g.,* MVIIA, GVIA, MVIIC are available from Bachem, Torrance, CA; SVIB is available from Peninsula Laboratories, Belmont, CA). Omega-conopeptides can also be synthesized by conventional solid phase methods, such as have been described (Olivera, B., *et al., Biochemistry* 23:5087-5090 (1984); U.S. Patents 5,051,403 and 5,364,842). The three disulfide linkages in the peptides may be formed by air oxidation in the presence of dithiothreitol (DTT) or optionally other thiol containing compounds (*e.g.*, cysteine, glutathione), and further purified according to procedures described elsewhere (WO 93/13128).

C. Omega-Conopeptide Formulations

Solutions of SNX-111 in which the peptide concentration is less than about 0.1 mg/ml oxidize rapidly when dissolved in water, saline, or any of a number of buffers used in the art of peptide chemistry. Such oxidation results in a reduction or loss of biological activity. In accordance with one aspect of the present invention, it has been discovered that omega-conopeptides can be significantly stabilized in solution by preventing oxidation of methionine residues present in the peptide structure. In particular, SNX-111, which contains a methionine at position 12, is approximately 10-fold less potent in binding to omega-conopeptide MVIIA binding sites when its methionine is present in the sulfoxy form.

In experiments carried out in support of the present invention, it was found that SNX-111 oxidation can be prevented by addition of carboxylic acid (lactate) buffer to the peptide solution. Solutions of SNX-111 ranging from 0.01-0.1 mg/ml are stable at 45°C for several weeks when stabilized with lactate (150 mM, pH 4-4.5). In addition, buffers that contain 50 μg/ml methionine are also effective in stabilizing SNX-111. Here, either 150 mM lactate buffer or acidified saline (pH 4-4.5) can be used to buffer the solution.

Such stable omega-conopeptide formulations are useful for storage or for use in methods that involve slow infusion of compound. The solution may be administered directly or neutralized prior to administration, according to the particular route of administration in which the formulation is used. For example, approximately 10 μl of SNX-111 in lactate buffer (150 mM, pH 4-4.5) has been administered directly into rat spinal cords (intrathecally) without noticeable untoward effects. Figure 10 shows the results of experiments demonstrating that SNX-111 administered as a methionine-lactate formulation is as effective as SNX-111 alone in reducing neuropathic pain.

Additional formulations may include means for enhancing permeation of the conopeptide through meningeal tissues which may surround the damaged or target nerve. Means for enhancing transport of compound are known in the art and may include encapsulating the conopeptide in liposomal membranes, addition of a surfactant to the composition, addition of an ion-pairing agent, and the like. Transmeningeal transport may also be facilitated by administering to the subject a hypertonic dosing solution effective to disrupt meningeal barriers, according to methods well known in the art. Alternatively, trans meningeal or transcutaneous transport may be further facilitated by modifying the primary sequence of omega-conopeptide, for example by substituting neutral amino acid sidechains or hydrophobic moieties for cationic residues.

III. Treatment Methods

A. Medicaments for Inhibiting Progression of Neuropathic Pain Disorders

It is the discovery of the present invention that progression of neuropathic pain disorders can be retarded in a subject exhibiting early stage symptoms of neuropathic pain, by administering to the subject an active N-type VSCC blocking omega conopeptide having the physical and pharmacological characteristics described above, particularly when such administering is localized to the neuropathic site.

This section describes the various modes of administration that can be used in preventing progression of neuropathic pain disorders. This section also describes an exemplary model neuropathic pain disorder characterized by allodynia (sensitivity to weak stimulus) in which an omega conopeptide medicament is used to prevent progression of the disorder.

1. Inhibiting Progression of Neuropathic Pain

The rat allodynia model described in Example 5 is an exemplary model of a neuropathic pain disorder. This model provides an example of how medicaments of the present invention may be used to inhibit progression of neuropathic pain. In addition, the model has been used to evaluate interventions and therapeutics effective in inhibiting the progression of neuropathic pain.

In the model, after experimental ligation of a nerve, neuropathic pain symptoms increase over days 1-7 after the nerve insult, with a plateau of pain response thereafter. An active omega conopeptide selected in accordance with the principles outlined in Section II above is assessed for its ability to inhibit or prevent such progression of neuropathy during days 1-7, by administering the compound to the test subject just before, during or after nerve insult. Threshold pain responses are measured in a standard paradigm on days 1-7, or longer, after the ligation insult. Such omega-conopeptide administration is carried out according to any of the methods described in sub-section 2, below.

In this exemplary model, inhibition of progression of the neuropathic pain disorder is observed when there is a heightened threshold to pain stimulus during days 1-7 or where there is a prolongation of time to plateau, as compared to control neuropathic animals.

Efficacious dosages and formulations determined in this model may be extrapolated to equivalent large animal and human dosages, according to methods well known in the art.

2. Modes of Administration of Active Omega-conopeptides for Inhibiting Progression of Neuropathic Pain Disorders

This section describes various modes of administration that can be used to deliver active omega-conopeptides to the site of progression of neuropathic pain, in order to inhibit such progression, in accordance with the present invention.

Perineural Administration. Medicaments containing N-type VSCC blocking omega conopeptides can be administered perineurally to prevent progression of neuropathic pain disorders. Here, the compounds are administered either directly to the affected nerve, or to affected skin regions characterized by proliferation of neurite outgrowth subsequent to nerve damage. For administration to the affected skin regions, the compound may be delivered topically, either directly or with the use of a transdermal applicator.

Alternatively, the medicament may be injected subdermally to the region of the nerve. In another form of perineural administration, a cuff around the damaged nerve, where the cuff is formed of a biodegradable matrix which includes a therapeutic N-type calcium channel blocking compound. Alternatively or in addition, the therapeutic compound can be placed in close proximity to the damaged nerve by conjugating the compound to or coating the compound on a nerve splint designed for repairing damaged nerves. Examples of such nerve splints are provided by U.S. Patents 4,534,349 and 4,920,962. Perineural delivery may also be effected by incorporating active omega conopeptides into suture materials, such as collagen-based suture material described in U.S. Patent 5,308,889. This material is then used to suture damaged tissues, such as due to trauma or surgery.

The compound can also be delivered to perineural sites via transdermal delivery. Generally, transdermal delivery involves the use of a transdermal "patch" which allows for slow delivery of compound to a selected skin region. For transdermal delivery of omega-conopeptides, transdermal delivery may preferably be carried out using iontophoretic methods, such as described in U.S. Patent 5,032,109 (electrolytic transdermal delivery system), and in U.S. Patent 5,314,502. It may be desirable to include permeation enhancing substances, such as fat soluble substances (*e.g.*, aliphatic carboxylic acids, aliphatic alcohols), or water soluble substances (*e.g.*, alikane polyols such as ethylene glycol, 1,3-propanediol, glycerol, propylene glycol, and the like). Alternatively, U.S. Patent 5,362,497 describes a "super water-absorbent resin" that can be added to transdermal formulations to further enhance transdermal delivery. Examples of such resins include, but are not limited to, polyacrylates, saponified vinyl acetate-acrylic acid ester copolymers, cross-linked polyvinyl alcohol-maleic anhydride copolymers, saponified polyacrylonitrile graft polymers, starch acrylic acid graft polymers, and the like. Such formulations may be also be used in occluded dressings to the region of interest.

For delayed release, the N-type VSCC blocking compound may be included in a pharmaceutical composition formulated for slow release, such as in microcapsules formed from biocompatible polymers known in the art. For continuous release of peptides, the peptide may be covalently conjugated to a water soluble polymer, such as a polylactide or biodegradable hydrogel derived from an amphipathic block copolymer, as described in U.S. Patent 5,320,840. Collagen-based matrix implants, such as described in U.S. Patent 5,024,841, are also useful for sustained delivery of peptide therapeutics. Also useful, particularly for subdermal slow-release delivery to perineural regions, is a composition that includes a biodegradable polymer that is self-curing and that forms an implant *in situ*, after delivery in liquid form. Such a composition is described, for example in U.S. Patent 5,278,202.

Although dosages will vary according to the physical characteristics of the individual patient, a dose should be selected to provide delivery of between about 1 and 100 μg/g tissue in the affected region.

Intrathecal Administration. Medicaments of the invention may also be administered to those regions of the spinal cord, such as to dorsal horn regions at affected vertebral levels, where polysynaptic relay of pain sensation occurs. This type of local application can be effected by intrathecal administration, as described in WO 93/13128.

Active omega conopeptides can be administered intrathecally by slow infusion. Known in the art are a number of implantable or body-mountable pumps useful in delivering compound at a regulated rate. One such pump described in U.S. Patent 4,619,652 is a body-mountable pump that can be used to deliver compound at a tonic flow rate or in periodic pulses. When the active omega conopeptide MVIIA is administered to humans via a continuous flow intrathecal route, doses in the approximate range of 0.1-500 ng/kg/hr may be required in order to achieve a therapeutic concentration of the compound.

Epidural Administration. Delivery to spinal cord regions can also be by epidural injection to a region of the spinal cord exterior to the arachnoid membrane. It is further appreciated that for administration of omega conopeptides via this route, it may be advantageous to add to the conopeptide composition means for enhancing permeation of the conopeptide through meningeal membranes. Such means are known in the art and include liposomal encapsulation, or addition of a surfactant or an ion-pairing agent to the peptide composition. Alternatively, or in addition, increased arachnoid membrane permeation can be effected by administering a hypertonic dosing solution effective to increase permeability of meningeal barriers.

According to another aspect of the invention described herein, epidural administration of active omega conopeptides can also be carried out by administering the omega-conopeptides epidurally without addition of a membrane permeabilizing agent. This aspect of the invention is discussed in Section IIIB, below.

Intravenous Administration. Intravenous administration of active omega conopeptides can be used to achieve therapeutic concentrations of compound at perineural regions and/or at spinal synapses, in accordance with the treatment principles described above. In order to achieve effective intraspinal levels of omega conopeptide, human doses equivalent to a dose range of 0.1-15 mg/kg in a rat may be required. In addition, the compound may be administered by continuous intravenous infusion at at rate of 1-10 mg/kg/hr to achieve therapeutic levels.

### B. Medicaments for Treatment of Pain by Epidural Administration

It is the further discovery of the present invention that analgesia can be produced by epidural administration of an omega conopeptide, without the addition of membrane permeabilizing agents to the composition. This finding was unexpected, based on the hydrophilic, charged properties of omega conopeptides, and the relatively low permeability of meningeal membranes surrounding the epidermal space to compounds having such physicochemical properties.

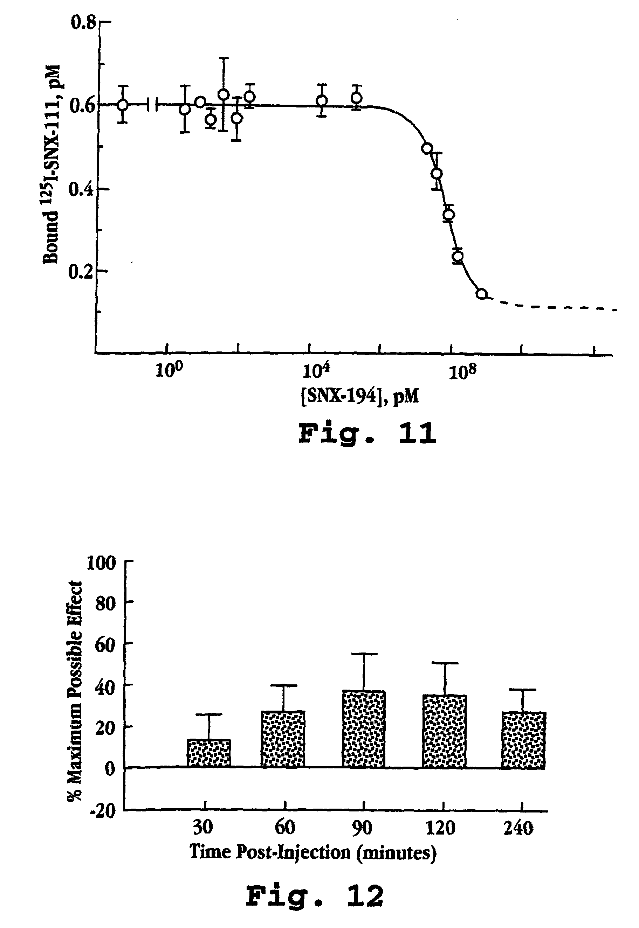

This unexpected result may be explained in part by results of experiments carried out in support of the present invention from which it has been determined that spinal meningeal membranes possess specific, relatively low affinity binding sites for omega conopeptides (Example 3, Figure 11). Without subscribing to a particular mechanistic theory, such binding may provide a rationale for the observed results *in vivo*. The competition binding curve shows low non-specific binding (approximately 20%), and a Hill coefficient close to one, consistent with binding to a single low-affinity

site. The calculated $IC_{50}$ value is 41 µM.

In experiments carried out in support of the invention, it is now shown that active omega-conopeptides, as defined above, can produce analgesia in subjects when these compounds are administered epidurally at doses that are equivalent to or just slightly higher than an effective intrathecal (intraspinal) dose of the same compound. Effective intrathecal doses determined in rats subjected to the rat allodynia model of peripheral neuropathy described in Example 5 range from 0.1-0.3 µg (SNX-239, SNX-159, SNX-273, SNX-279, SNX-111). When given by continuous intrathecal infusion, the total dose of active omega conopeptide required to produce analgesia is about the same (2.4 µg SNX-111) as the bolus dose.

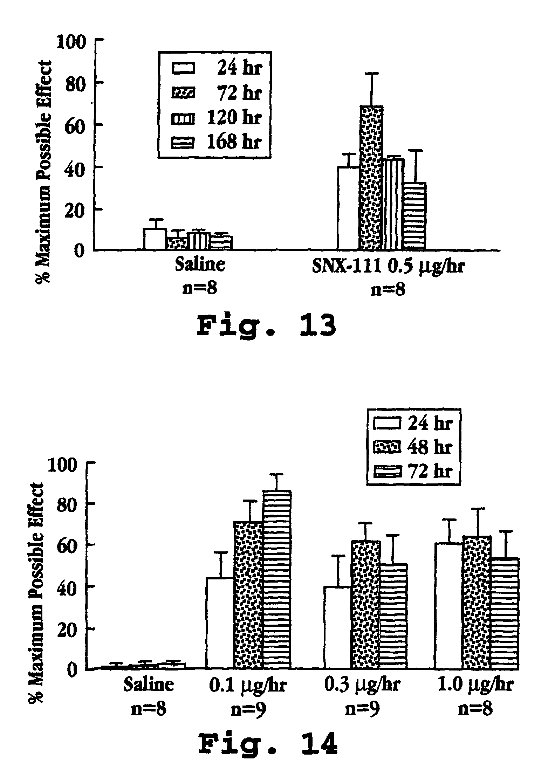

In exemplary experiments carried out in support of the present invention an epidural dose of 30 µg SNX-111 is effective to produce analgesia in a rat subjected to peripheral neuropathy (Figure 12). This dose is within the effective range (3-30 µg) of an intrathecal dose of SNX-111 in the same experimental model. More strikingly, active omega conopeptide SNX-111 produced analgesia at comparable doses when administered by constant intrathecal infusion at 0.5 µg/hr (Figure 13) or constant epidural infusion at 0.1, 0.3 or 1.0 µg/hr (Figure 14). When the compound is administered in this manner, it is in prolonged contact with the epidural region. From these data it can be seen that such prolonged contact is desirable in epidural administration. Other useful ways of providing prolonged contact, such as via sustained release formulations, are known in the art and are disclosed in Section IIIA2, above.

In practicing the invention, a subject experiencing pain is given, via standard epidural administration procedures, an effective analgesic dose of an active omega conopeptide, as that term is defined in Sections I and II, above. By "within the range" is meant that the dose should be within about ± an order of magnitude of the effective intrathecal dose, and more preferably within ± 0.5 log units of the dose. Thus, in the example described above, where a total dose of 1-3 µg SNX-111 is effective intrathecally to produce analgesia in a rat, the range for epidural administration will be from about 0.1-30 µg.

In humans, SNX-111 has been found to provide analgesia when given as a continuous infusion intrathecally at a dose of between 0.3 and 300 ng/kg/hr over a period of 24 hours. While the precise dose will depend on the size and other physiological attributes of the subject, a human epidural dose will be within the range of dose required for analgesia when the compound is given intrathecally. Based on the foregoing, an effective epidural dose in humans could be expected to be in the range of 0.3-3000 ng/kg/hr.

While it is preferred that the epidural dose be given by continuous infusion for at least 24 hours, this and dosage parameters will be adjusted by the clinician, according to the needs of the particular patient, within the framework of standard clinical protocols for regulating pain medication. Further information concerning dosage can be determined with reference to the standard animal models known in the art, in accordance with standard pharmacokinetic principles.

In addition to the rat allodynia model of peripheral neuropathy described above, other standard animal models, such as the Rat Tail-Flick Model or the Rat Formalin Model can be used to determine efficacy and to estimate doses for treating nociceptive pain using epidural administration of an active omega-conopeptide. Generally, anti-nociceptive doses are lower than the doses required to produce analgesia in patients suffering from neuropathic pain.

Stabilized formulations, as described in Section IIC, above, are useful in manufacturing the medicaments for epidural administration, and thus form part of the invention. While it may be desirable to neutralize the solutions prior to administration, formulations utilizing lactate or acidified saline as excipient may also be administered directly via acute epidural or intrathecal bolus injection or by other routes for which acidified excipients are appropriately used. Neutralization, if required, can be accomplished by dilution into a pharmaceutically acceptable neutralizing excipient buffer, just prior to administration to the subject.

The following examples are intended to illustrate various characteristics of the method of the invention, but are in no way intended to limit the scope of the invention.

Example 1

Calcium-Channel Antagonist Activity: Inhibition of Ionic Currents

Ionic currents through calcium channels were examined in cells that were voltage-clamped by a single patch-clamp electrode. These whole-cell patch-clamp studies were performed on N1E115 mouse neuroblastoma cells, although a variety of cell types, including human neuroblastoma cell line IMR-32, can also be used for determining calcium antagonist activity.

A. Current Measurement Methods

Bath solutions were designed to allow measurement of calcium channels in the absence of other channels. Solutions were prepared according to standard protocols and contained 80 mM N-methyl-D-glucamine (NMDG) (as a sodium replacement), 30 mM tetraethylammonium chloride (TEAC1) (to block potassium currents), 10 mM $BaCl_2$ (as a charge-carrier through the calcium channels), and 10 mM HEPES at pH 7.3. Some solutions also contained 2 mM quinidine (to block potassium currents) and 3 $\mu$M tetrodotoxin (to block sodium currents). Normal bath saline was (mM): 140 NaCl, 10 glucose, 3 KCl, 2 $CaCl_2$, 1 $MgCl_2$, 10mM HEPES pH 7.3. Intracellular solutions contained 150 mM CsCl, 0.5 mM $CaCl_2$, 5 mM EGTA, 5 mM $MgCl_2$, 2 mM $K_2$ATP at pH 7.3-7.4. Bath saline and all internal solutions were filtered before use.

Pipets were made from Corning 7052 glass (Garner Glass Company, Claremont, CA 91711), coated with Sylgard (Dow Corning, Midland, MI 48640) and fire-polished before use. Bubble numbers were typically 5 to 6, with pipet resistances typically 2-5 Mohms. Corning 8161, Kimble, and other glasses were also used without noticeable effect on the calcium currents observed.

Recordings were carried out at room temperature with an Axopatch 1-C amplifier (Axon Instruments, Foster City, CA 94404) and analyzed with pCLAMP software (Axon Instruments). Data were filtered at 1000 Hz for a typical sampling rate of .1 kHz; analysis was performed on-screen with print-out via a Hewlett-Packard LaserJet Printer (Hewlett-Packard, Palo Alto, CA 94306).

The typical experiment was conducted as follows: after seal formation followed by series resistance compensation and capacitive transient cancellation, a voltage clamp protocol was performed wherein the cell potential was stepped from the holding potential (typically -100 mV) to test potentials that ranged from -60 mV to +20 mV in 10 mV increments. The cell was held at the holding potential for 5 seconds between pulses.

B. Current Inhibition Measurement

Calcium current traces were taken from an N1E-115 mouse neuroblastoma cell, as shown in Figure 7. The figure is read from left to right in time, with downward deflections of the trace indicating positive current flowing into the cell. Currents were elicited by a voltage step from 100 mV to -10 mV. The cell was bathed in saline with sodium replaced by NMDG and 10 mM $Ba^{++}$ instead of 2 mM $Ca^{++}$. Potassium currents were blocked by TEA in the bath and $Cs^+$ in the pipet solution.

The three traces in Figure 7, labeled b-d, show decreasing calcium currents, with increasing MVIIA omega-conopeptide concentrations of 10 nM (b), 50 nM (c), and 200 nM (d).

Example 2

Omega-Conopeptide Binding to Omega-conopeptide Binding Sites in Synaptosomal Membranes

A. Preparation of Mammalian-Brain Synaptosomes and Synaptosomal Membranes

Synaptosomes were prepared from rat whole brain or hippocampal region of brain. Rats were sacrificed, and forebrains were removed and transferred to 10 ml ice-cold 0.32 M sucrose containing the following protease inhibitors (PI): 1 mM EGTA; 1 mM EDTA; 1 uM pepstatin; 2 uM leupeptin. Brains were homogenized using a motor-driven Teflon-glass homogenizer (approx. 8 passes at 400 rpm). Homogenates from 4 brains were pooled and centrifuged at 900 $\times$ g for 10 minutes at 4°C. Supernatants were then centrifuged at 8,500 $\times$ g for 15 minutes. Resulting pellets were resuspended in 10 ml each ice-cold 0.32 M sucrose plus PI with vortex mixing. The suspension was then centrifuged at 8,500 $\times$ g for 15 minutes. Pellets were resuspended in 20 ml ice-cold 0.32 M sucrose plus PI. The suspension (5 ml/tube) was layered over a 4-step sucrose density gradient (7ml each: 1.2 M sucrose, 1.0 M sucrose, 0.8 M sucrose, 0.6 M sucrose; all sucrose solutions containing PI). Gradient tubes were centrifuged in a swinging bucket rotor at 160,000 $\times$ g for 60 minutes at 4°C. The 1.0 M sucrose layer plus the interface between the 1.0 and 1.2 M sucrose layers were collected and diluted with ice cold deionized water plus PI to yield a final sucrose concentration of 0.32 M. The resulting suspension was centrifuged at 20,000 $\times$ g for 15 minutes. Pellets were then resuspended in 5 ml ice-cold phosphate buffered saline plus PI. The resulting rat brain synaptosomes were then aliquoted and stored in a liquid nitrogen containment system.

Prior to use in binding assays, synaptosomes were thawed and diluted with 3 volumes of ice cold deionized water plus PI. This suspension was homogenized using a PT 10-35 Polytron (setting 6) for two 10-second bursts. The homogenate was centrifuged at 40,000 $\times$ g for 20 minutes at 4°C. The resulting pellets were resuspended in about 5 ml of ice cold phosphate buffered saline plus PI. The resulting brain synaptosomal membrane preparation was aliquoted and stored at -80°C until use. Protein concentration of the membrane preparation was determined using Bradford reagent (BioRad), with bovine serum albumin as standard.

B. Competitive Displacement Binding Assay

1. Competitive Displacement of OCT MVIIA. Rat brain synaptosomal membranes prepared as described in Part A were suspended in a binding buffer consisting of 20 mM HEPES, pH 7.0, 75 mM NaCl, 0.1 mM EGTA, 0.1 mM EDTA, 2μM leupeptin, .035 μg/ml aprotinin, and 0.1% bovine serum albumin (BSA). [125I]-MVIIA (SNX-111) OCT (25-30,000 cpm, approximately 1500-2000 Ci/mmol) and test compound were aliquoted into polypropylene tubes, in the absence or presence of 1 nM MVIIA (SNX-111) OCT to determine non-specific binding. The membrane suspension was diluted and aliquoted last into the test tubes, such that each assay tube contained 10 μg membrane protein and the total volume was 0.5 ml. After incubation for 1 hour at room temperature, tubes were placed in an ice bath, then filtered through GF/C filters (Whatman), which were pre-soaked in 0.6% polyethyleneimine and prewashed with wash buffer (20 mM HEPES, pH 7.0, 125 mM NaCl, 0.1% BSA) using a Millipore filtration system. Just prior to filtration, each assay tube received 3 ml ice-cold wash buffer. The filtered membranes were washed with two 3 ml volumes of ice-cold wash buffer, dried, and filter-bound radioactivity was measured in a Beckman gamma counter (75% counting efficiency).

IC$_{50}$ values were computed from competitive displacement line fit curves generated by a 4-parameter logistic function. These values represent the concentration of test compound required to inhibit by 50% the total specific binding of [125I]-MVIIA (SNX-111) OCT to rat brain synaptosomal membranes, where specific binding is defined as the difference between binding of [125I]-MVIIA (SNX-111) OCT in the absence and presence of excess (1 nM) unlabelled MVIIA OCT. Non-specific binding is that binding of radiolabeled compound which is measured in the presence of excess unlabeled MVIIA OCT. Such values serve as approximations of the relative affinities of a series of compounds for a specific binding site.

2. Competitive Displacement of OCT SVIB. Rat brain synaptosomal membranes were prepared as described above. OCT SVIB was radiolabeled by iodination with 125I-iodine by the Iodogen reaction. Displacement binding of radiolabeled SVIB on rat brain synaptosomal membranes was carried out as in Example 4B. IC$_{50}$ values and relative potency values were calculated as described below. Tables 2 and 3 show the relative potency values for omega-conopeptides examined, and the ratio of relative potencies of the compounds for the OCT MVIIA site and to the SVIB binding site.

The binding constant (K$_i$) for each test substance was calculated using non-linear, least-squares regression analysis (Bennett, G.J. and Xie, Y.-K., *Pain* 33:87-107 (1988)) of competitive binding data from 2 assays performed in duplicate on separate occasions. The relationship between K$_i$ and IC$_{50}$ (concentration at which 50% of labeled compound is displaced by test compound is expressed by the Cheng-Prusoff equation:

$$K_i = IC_{50}/(1 + [L]/K_d)$$

where IC$_{50}$ is the concentration of test substance required to reduce specific binding of labeled ligand by 50%; [L] is the concentration of [125I]-MVIIA (SNX-111) OCT used in the experiment; and K$_d$ is the binding constant determined for binding of [125I]-MVIIA (SNX-111) OCT to rat brain synaptosomal membranes in saturation binding experiments. Table 3 summarizes computed IC$_{50}$ for various omega-conopeptides for the MVIIA binding site of rat brain synaptosomal membranes. Relative potency for displacement of binding is calculated as a ratio of the IC$_{50}$ of the test compound and the IC$_{50}$ of the reference compound. The reference compound is generally the unlabeled equivalent of the labeled ligand. Calculation of relative potency is as follows:

$$[log \ (relative \ potency)] = log \ (IC_{50(ref)}) - log(IC_{50(tert)})$$

Example 3

Binding to Meningeal Membranes

Meningeal membranes were obtained from the spinal cords (T3 to L5) of *Macaque nemestrina* monkeys. The spinal cords were removed and incisions were made simultaneously through all three meningeal layers along the ventral surfaces. Together, the dura, arachnoid, and pia mater were carefully collected from each spinal cord, preserving their normal anatomic relationships.

Frozen spinal pia-arachnoid membranes were minced, suspended in 5 ml of deionized water containing protease inhibitors (1 mM EDTA, 1 EGTA, 1 μM pepstatin, 2 μM leupeptin), homogenized with a Polytron (PT-10-35, speed 6) for 10 seconds, and centrifuged at 40,000 g for 20 min. The membrane pellet was resuspended in 5 ml of 0.05 M phosphate buffer, pH 7.4, containing protease inhibitors and stored in liquid nitrogen until used. The protein concentration in the membrane preparation was determined using the Bradford Reagent (Bio-Rad) with bovine serum albumin (BSA) as a standard. MVIIA OCT was radiolabeled with $^{125}$I-iodine by reaction with Iodogen™, essentially according to the method of Ahmad and Miljanich (Ahmad, S.N. and Miljanich, G.P., *Brain Research* 453:247-256 (1988) Binding assays were carried out at room temperature in 12 × 75-mm polypropylene tubes. The binding buffer contained 20 mM Hepes, pH 7.2, 75 mM NaCl, 0.1 mM EDTA, 0.1 mM EGTA, 2.0 μM leupeptin, 0.5 U aprototinin, and 0.1% BSA. The binding mixture contained 5 μg of meningeal membranes in 0.2 ml, $^{125}$I-SNX-111 in 0.1 ml, and 0.2 ml binding buffer to give a final volume of 0.5 ml per tube. $^{125}$I-SNX-111 competition binding curves were generated by adding various concentrations of SNX-194 ([Nie[12] SNX-111) to the assay tubes. Non-specific binding was determined in the presence of 500 μM of SNX-194. The reaction was terminated by transferring the assay tubes to an ice-cold water bath and diluting each sample with 3 ml of chilled washing buffer (20 mM Hepes, pH 7.2, 125 mM NaCl, 0.1% BSA). Membrane-bound radioactivity was separated on a Millipore manifold filtration system using glass fiber filters which were presoaked in 0.6% polyethyleneimine. Filters were washed twice (3 ml) with ice-cold washing buffer and counted in a Beckman gamma counter (Model G-500) at 75% counting efficiency. Data were fitted to a four-parameter logistic function:

$$\text{Effect} = E_o + (E_i - E_o) \bullet 1/\ 1+ (K/L)^h$$

where $E_o$ = effect at 0 dose, $E_i$ = effect at infinite dose, $K = IC_{50}$, $L$ = concentration of competing ligand, and $h$ = slope. Parameter values were calculated by computer (Hewlett-Packard 9000) using a nonlinear, least-squares curve-fitting algorithm.

Example 4

Inhibition of Electrically Stimulated Contractions of Guinea Pig Ileum

Guinea pigs (300-400 gms) were decapitated and the ileum removed. A section of ileum about 6 cm from the caecum was placed immediately into Krebb's modified buffer maintained at 37°C in a water bath, and aerated with a mixture of 95% $O_2$ and 5% $CO_2$. The buffer contains: KCl, 4.6 mM; $KH_2PO_4$, 1.2 mM; $MgSO_4$, 1.2 mM; Glucose, 10.0 mM; NaCl 118.2 mM; $NaHCO_3$, 24.8 mM; $CaCl_2$, 2.5 mM.

Small pieces of ileum were cut and pulled over a glass pipette, scored and the longitudinal muscle removed. Each piece was attached to an electrode at one end and to a force transducer at the other end. The preparation was lowered into an organ bath maintained at 37°C and aerated with $O_2:CO_2$. The resting tension was set at 1 gm, and the tissue was stimulated at 30-50V with a duration of 4.5 msec per stimulation.

Baseline responses (contractions) were recorded for 10-15 min. and aliquots (100 ml) of drug were added to the bath until inhibition occurred. Following testing, tissues were washed until original response magnitude was achieved.

Example 5

Rat Allodynia Model of Peripheral Neuropathy

The animal model of neuropathy described herein resembles the human condition termed causalgia or reflex sympathetic dystrophy (RSD) secondary to injury of a peripheral nerve. This condition is characterized by hyperesthesia (enhanced sensitivity to a natural stimulus), hyperalgesia (abnormal sensitivity to pain), allodynia (widespread tenderness, characterized by hypersensitivity to tactile stimuli), and spontaneous burning pain. These symptoms generally increase or progress with time.

The experimental model described below is designed to reproducibly injure peripheral nerves (spinal nerves L5 and L6) of rats. Rats develop a hyperesthetic state which can be measured. Here, allodynia was measured by stimulation of neuropathic rat hindlimb using wire hairs having graded degrees of stiffness. Analgesic compounds reverse the heightened sensitivity such animals exhibit to the stimulus. Data from such a model are generally expressed as percent maximum effect, where the maximum effect indicates a complete reversal of surgically induced allodynia, or relative insensitivity to a maximum stimulus provided by a 15 gram "hair" or "wire."

For intrathecal administration of compounds, male Sprague-Dawley rats (250-350 gm) were prepared with chronic lumbar intrathecal catheters inserted under halothane anesthesia (Yaksh, T.L., and Rudy, T.A., *Physiol. Behav.* 17:1031-1036 (1976)).

Neuropathogenic surgery: Animals were anesthetized with pentobarbital sodium, placed in a prone position and the left paraspinal muscles were separated from the spinous processes at the $L_4$-$S_2$ levels, as described by Kim and Chung (Kim, S.H. and Chung, J.M., *Pain* 50:355-363 (1992)). The left L5 and L6 nerve roots were exposed and tightly ligated with 6-0 surgical silk suture.

Animals were allowed to recover from the neuropathogenic surgery and nociceptive responses were measured daily until the animals exhibited consistent signs of mechanical allodynia (approximately 7-10 days). For epidural administration of drugs, the animals were then implanted with indwelling spinal (lumbar) epidural catheters according to the procedure of Durant and Yaksh (Durant, P.A.C., and Yaksh, T.L., *Anesthesiology* 64:43-53 (1986)), with minor modifications. SNX-111 or vehicle (saline) was administered either by bolus injection one-day after catheter placement or by continuous, constant-rate infusion initiated at the time of catheter implantation. For bolus injection, SNX-111 was administered in a volume of 30 μl followed by 10 μl saline to flush the line. For continuous epidural infusion, catheters were connected to indwelling mini-osmotic pumps and test solutions were delivered at a rate of 0.5-1.0 μl/hr. Mechanical allodynia thresholds were measured immediately before and at fixed intervals during or after SNX-111 administration.

For intrathecal administration of drugs, the lumbar subarachnoid space was catheterized with saline-filled polyethylene (PE-10) tubing as described by Yaksh and Rudy (Yaksh, T.L., and Rudy, T.A., *Physiol. Behav.* 17: 1031-1036 (1976)). The catheter was anchored with stay sutures to the adjacent muscle tissue where it emerged from the cisterna magna. For bolus injection, test compound was dissolved in saline and administered in a volume of 10 μl through the intrathecal catheter, followed by 10 μl saline to flush the catheter line. Continuous infusion was carried out as described above.

Animals were given at least 3 days to recover from catheterization before assessing mechanical allodynia thresholds. Allodynia was typically observed to occur beginning 1-2 days post-surgery and continued for as long as 45 days. Animals showing motor deficits were excluded from further study.

To assess the threshold of a non-noxious stimulus required to produce a left hind paw withdrawal (allodynia), Von Frey filaments (Stoelting, Wood Dale, IL) of graded stiffness (ranging from 0.4-15 grams), were systematically applied to the surgically treated plantar of the hind paw. The hair was held against the surface with sufficient force to cause slight bending and held for 6-8 seconds. If there was no response, the next stiffer hair was tested. Evocation of a brisk withdrawal response was cause to test the next lower stimulus intensity. This paradigm was repeated according to a statistical method (Dixon, W.J., *Ann. Rev. Pharmacol. Toxicol.* 20:441-462 (1976)) to define the 50% response threshold. Allodynia was evidenced by a threshold less than 3 grams (referring to the hair stimulus intensity) exhibited by all surgically treated animals.

The mechanical stimulus upper limit for threshold testing was set at 15 grams. %MPE values near 100 indicate normal mechanical thresholds (i.e. at or near 15 gms); values near 0 indicate allodynia. After completion of testing, animals were killed and the vertebral columns were dissected to confirm the position of the catheters and to examine the condition of the catheter tips. Catheters were then removed and flushed with saline to detect leaks and confirm patency. Animals were excluded from the study if the catheters were obstructed or not appropriately positioned in the epidural space.

Data obtained in this model are expressed as percent maximum effect (%MPE) according to the equation below, where the maximum effect indicates a complete reversal of allodynia, or insensitivity to stimulus (maximum equals 15 gram hair cutoff). A baseline of zero indicates a mean sensitivity less than 3 grams.

$$\%MPE = \frac{NewThreshold\ (g)\ -\ BaselineTreshold\ (g)\ x\ 100}{15\ grams\ -\ BaselineTreshold}$$

According to this analysis, the higher the %MPE, the better the antinociceptive effect. Active conopeptides, including SNX-273 and SNX-279, block mechanical allodynia significantly in comparison to saline control. The apparent order of potency for suppression of allodynia is SNX-111=SNX-273 > SNX-279. This is consistent with the compounds' relative affinities at the SNX-111 binding site ($IC_{50}$'s: SNX-111, 8 pM; SNX-273, 8 pM; SNX-279, 40 pM).

Animals were also observed for the appearance of general motor dysfunction, as evidenced by inability to ambulate symmetrically and for any other overt signs of unusual activity. No effects on motor activity were observed in saline-treated animals; a dose-dependent tremor characteristic of SNX-111 administration was observed in animals given SNX-111.

A. Inhibition of Progression of Neuropathic Pain.

SNX-111 is administered to animals to assess prevention of development of neuropathy, as evidenced by reduction in development of mechanical and thermal allodynia. Animals are implanted with indwelling intravenous (right jugular vein) or spinal intrathecal catheters attached to subcutaneously implanted osmotic minipumps (Alza Corp., Palo Alto, CA) to provide continuous constant-rate infusion of compound or vehicle (acidified physiologic saline, pH 4-4.5). For chronic studies, animals are allowed twenty-four hours recovery time before being subjected to the surgical procedure described above to produce a peripheral nerve injury. Infusion of drug or vehicle is commenced one day before surgery. Doses of SNX-111 tested in initial studies are 100 µg/kg/hr i.v. (14 days) and 100 ng/kg/hr i.t. (14 days)

Mechanical allodynia is assessed each day for about forty (40) days, commencing the day prior to surgery using van Frey wire stimuli, as described above. Mean stimulus thresholds are compared over time between drug-treated and control (vehicle-treated) animals.

In addition, or in a separate set of treated animals, thermal stimuli are given as acute or persistent stimuli. Acute stimuli are administered by applying acetone to the plantar surface of the affected foot and counting brisk foot withdrawals in response to the application. Response to persistent stimuli is measured by placing the rat on a brass plate kept at neutral (30°C) or cold (5°C) temperature. After five minutes of adaptation, the cumulative duration of time that the rat holds its foot off the plate during a five minute observation period is measured. Data are analyzed as described above.

Example 6

Methionine-Lactate Buffer Formulations

Analgesic efficacy of spinally-administered SNX-111 was tested using a methionine-lactate buffer formulation in the paradigm detailed in Example 5. SNX-111 (10 µg/ml) and L-methionine (50 µg/ml) were dissolved in a vehicle comprised of sodium lactate (150 mM) adjusted to pH 4-4.5 with 250 mM lactic acid. This formulation was used to deliver 0.1 µg SNX-111 intrathecally, as described in Example 5 at 30, 60, 120 and 240 minutes after treatment with test or control compound. Figure 10 shows effects on mechanical allodynia of a single intrathecal bolus injection of 10 µl saline (open circles) or lactate buffer (150 mM) containing 50 µg/ml methionine with (closed squares) or without (closed triangles) 10 µg/ml SNX-111. Neither saline alone or methionine lactate control buffer alone was effective to suppress allodynia, whereas the SNX-111 formulation was effective in this regard. Moreover, it was observed that %MPE values for saline-treated controls were not significantly different from those of animals given methionine-lactate buffer alone.

Although the invention has been described with respect to particular embodiments, it will be apparent to those skilled that various changes and modifications can be made without departing from the invention.

**Claims**

1. Use of an N-type voltage-sensitive calcium channel blocking omega conopeptide **characterized by** an ability to (a) inhibit electrically stimulated contraction of the guinea pig ileum, and (b) bind selectively to omega conopeptide MVIIA binding sites present in neuronal tissue, as evidenced by the ability of the omega-conopeptide to displace MVIIA from said site, in the manufacture of a medicament for inhibiting progression of a neuropathic pain disorder, when the omega conopeptide is administered to a subject at the site of progression of nerve injury.

2. The use of claim 1, wherein the activities of the omega-conopeptide in inhibiting guinea pig ileum contraction and in binding to the MVIIA binding site are within the ranges of such activities of omega-conotoxins MVIIA and TVIA.

3. The use of claim 1 or claim 2, wherein said conopeptide is selected from the group consisting of TVIA/SNX-185, MVIIA/SNX-111, SNX-236, SNX-159, SNX-239, SNX-199, SNX-273, SNX-279, and derivatives thereof.

4. The use of any one of claims 1 to 3, wherein said medicament further includes an anti-oxidant effective to prevent methionine oxidation.

5. The use of any one of claims 1 to 4, wherein said activity to bind selectively to the omega conopeptide MVIIA binding site is further evidenced by a selectivity ratio of binding affinity for said MVIIA binding site to binding affinity for a site 2 omega conopeptide binding site which is within the range of selectivity ratios determined for omega conopeptides MVIIA/SNX-111, SNX-199, SNX-236, SNX-239 and TVIA/SNX-185.

6. The use of any one of claims 1 to 5, wherein the medicament is used to inhibit progression of neuropathic pain at an injured nerve site, and is administered perineurally at the site of nerve injury.

7. A stable omega conopeptide formulation comprising an omega conopeptide and an anti-oxidant composition effective to prevent methionine oxidation.

8. The formulation of claim 7, wherein the anti-oxidant composition includes a carboxylic acid buffer.

**9.** The formulation of claim 8, wherein the carboxylic acid buffer is lactate buffer.

**10.** The formulation of claim 7, wherein the anti-oxidant is methionine.

**11.** The formulation of claim 7, wherein the anti-oxidant composition includes lactate and methionine.

**12.** An omega-conopeptide SNX-273 having the sequence:

CKGKGAKCSRLAYDCCTGSCRSGKC.

**13.** An omega conopeptide SNX-279 having the sequence CKGKGAKCSRLM(0)YDCCTGSCRSGKC, where M(O) indicates an oxidized methionine residue.

**14.** Use of an N-type voltage-sensitive calcium channel blocking omega conopeptide in the manufacture of a medicament for producing analgesia in a subject by epidural administration to the subject, comprising an omega conopeptide which is effective (a) to inhibit electrically stimulated contraction of the guinea pig ileum, and (b) to bind selectively to omega conopeptide MVIIA binding sites present in neuronal tissue, wherein the activities of the omega conopeptide in inhibition of guinea pig ileum and in binding to the MVIIA binding site are within the ranges of such activities of omega-conotoxins MVIIA and TVIA, and wherein said epidural administration is via a spinal epidural route over a period of time such that said conopeptide is in prolonged contact with the epidural region, at a dosage that is in the range of an effective intrathecal analgesic dose administered over an equivalent period of time.

**15.** The use of claim 14, wherein said epidural administering is via continuous infusion.

**16.** The use of claim 14 or 15, wherein said prolonged contact is effected by administering said conopeptide in a sustained release formulation.

**17.** The use of any one of claims 14 to 16, wherein said conopeptide is selected from the group consisting of MVIIA/SNX-111, TVIA/SNX-185, SNX-236, SNX-159, SNX-239, SNX-199, SNX-273, SNX-279, and derivatives thereof.

**18.** The use of any one of claims 14 to 17, wherein said activity to bind selectively to omega conopeptide MVIIA binding sites is further evidenced by a selectivity ratio of binding to said MVIIA binding site to binding at a site 2 omega conopeptide binding site which is within the range of selectivity ratios determined for omega conopeptides MVIIA/SNX-111, SNX-199, SNX-236, SNX-239 and TVIA/SNX-185.

**Revendications**

**1.** Utilisation d'un omega-conopeptide bloquant les canaux calciques sensibles aux hautes tensions de type N **caractérisé par** une aptitude à (a) inhiber la contraction stimulée électriquement de l'iléon de cobaye, et (b) se lier sélectivement aux sites de liaison de l'omega-conopeptide MVIIA présents dans un tissu neuronal, comme le montre l'aptitude de l'omega-conopeptide à déplacer MVIIA dudit site, dans la fabrication d'un médicament pour inhiber la progression d'une affection à douleur neuropathique, quand l'omega-conopeptide est administré à un sujet au site de progression d'une lésion nerveuse.

**2.** Utilisation selon la revendication 1 où les activités de l'omega-conopeptide dans l'inhibition de la contraction de l'iléon de cobaye et dans la liaison au site de liaison de MVIIA sont dans les domaines de telles activités d'omega-conotoxines MVIIA et TVIA.

**3.** Utilisation selon la revendication 1 ou la revendication 2 où ledit conopeptide est choisi dans le groupe consistant en TVIA/SNX-185, MVIIA/SNX-111, SNX-236, SNX-159, SNX-239, SNX-199, SNX-273, SNX-279 et leurs dérivés.

**4.** Utilisation selon l'une quelconque des revendications 1 à 3 où ledit médicament inclut en outre un anti-oxydant efficace pour empêcher l'oxydation de la méthionine.

**5.** Utilisation selon l'une quelconque des revendications 1 à 4 où ladite activité pour se lier sélectivement au site de liaison de l'omega-conopeptide MVIIA est mise en évidence en outre par un rapport de sélectivité de l'affinité de liaison pour ledit site de liaison de MVIIA à l'affinité de liaison pour un site de liaison d'omega-conopeptide de type site 2 qui est situé dans le domaine des rapports de sélectivité déterminés pour les omega-conopeptides MVIIA/SNX-111, SNX-199, SNX-236, SNX-239 et TVIASNX-185.

6. Utilisation selon l'une quelconque des revendications 1 à 5 où le médicament est utilisé pour inhiber la progression d'une douleur neuropathique à un site nerveux lésé, et est administré par voie périneurale au site de lésion nerveuse.

7. Formulation d'omega-conopeptide stable comprenant un omega-conopeptide et une composition d'anti-oxydant efficace pour empêcher l'oxydation de la méthionine.

8. Formulation selon la revendication 7 où la composition d'anti-oxydant inclut un tampon acide carboxylique.

9. Formulation selon la revendication 8 où le tampon acide carboxylique est un tampon lactate.

10. Formulation selon la revendication 7 où l'anti-oxydant est la méthionine.

11. Formulation selon la revendication 7 où la composition d'anti-oxydant inclut un lactate et de fa méthionine.

12. Omega-conopeptide SNX-273 ayant la séquence :

CKGKGAKCSRLAYDCCTGSCRSGKC.

13. Omega-conopeptide SNX-279 ayant la séquence :

CKGKGAKCSRLM(0)YDCCTGSCRSGKC,

où M(O) indique un résidu méthionine oxydé,

14. Utilisation d'un omega-conopeptide bloquant les canaux calciques sensibles aux hautes tensions de type N dans la fabrication d'un médicament pour produire une analgésie chez un sujet par administration épidurale au sujet, comprenant un omega-conopeptide qui est efficace (a) pour inhiber une contraction stimulée électriquement de l'iléon de cobaye, et (b) pour se lier sélectivement aux sites de liaison de l'omega-conopeptide MVIIA présents dans un tissu neuronal, où les activités de l'omega-conopeptide dans l'inhibition de l'iléon de cobaye et dans la liaison au site de liaison de MVIIA sont dans les domaines de telles activités d'omega-conotoxines MVIIA et TVIA, et où ladite administration épidurale est réalisée par une voie épidurale spinale pendant une durée telle que ledit conopeptide est en contact prolongé avec la région épidurale, à une posologie qui est dans le domaine d'une dose analgésique intrathécale efficace administrée pendant une durée équivalente.

15. Utilisation selon la revendication 14 où ladite administration épidurale est réalisée par infusion continue.

16. Utilisation selon la revendication 14 ou 15 où ledit contact prolongé est réalisé par administration dudit conopeptide dans une formulation à libération prolongée.

17. Utilisation selon l'une quelconque des revendications 14 à 16 où ledit conopeptide est choisi dans le groupe consistant en MVIIA/SNX-111, TVIA/SNX-185, SNX-236, SNX-159, SNX-239, SNX-199, SNX-273, SNX-279 et leurs dérivés.

18. Utilisation selon l'une quelconque des revendications 14 à 17 où ladite activité pour se lier sélectivement à des sites de liaison de l'omega-conopeptide MVIIA est mise en évidence en outre par un rapport de sélectivité de la liaison audit site de liaison de MVIIA à la liaison à un site de liaison d'omega-conopeptide de type site 2 qui est dans le domaine des rapports de sélectivité déterminés pour les omega-conopeptides MVIIA/SNX-111, SNX-199, SNX-236, SNX-239 et TVIA/SNX-185.

EP 0 835 126 B1

**Patentansprüche**

1. Verwendung eines spahnungsempfindlichen, Calciumkanal-blockierenden Omega-Conopeptids vom N-Typ, **gekennzeichnet durch** die Fähigkeit,

   (a) die elektrisch stimulierte Kontraktion des Meerschweinchenileums zu hemmen, und
   (b) selektiv an im neuronalen Gewebe vorhandene Omega-Conopeptid-MVIIA-Bindungsstellen zu binden, was **durch** die Fähigkeit des Omega-Conopeptids bewiesen wird, MVIIA an dieser Stelle zu ersetzen,

   für die Herstellung eines Medikaments zur Hemmung des Fortschreitens der neuropathischen Schmerzerkrankung, wenn das Omega-Conopeptid einem Individuum an der Stelle verabreicht wird, an der die Nervenverletzung fortschreitet.

2. Verwendung nach Anspruch 1, wobei die Aktivitäten des Omega-Conopeptids, die Kontraktion des Meerschweinchenileums zu hemmen und an die MVIIA-Bindungsstelle zu binden, innerhalb der Bereiche solcher Aktivitäten der Omega-Conotoxine MVIIA und TVIA liegen.

3. Verwendung nach Anspruch 1 oder Anspruch 2, wobei das Conopeptid ausgewählt ist aus der Gruppe bestehend aus TVIA/SNX-185, MVIIA/SNX-111, SNX-236, SNX-159, SNX-239, SNX-199, SNX-273, SNX-279, und Derivaten davon.

4. Verwendung nach einem der Ansprüche 1 bis 3, wobei das Medikament weiterhin ein Antioxidans einschließt, das die Methioninoxidation wirksam verhindert.

5. Verwendung nach einem der Ansprüche 1 bis 4, wobei die Aktivität, selektiv an die Omega-Conopeptid-MVIIA-Bindungsstelle zu binden ferner durch das Selektivitätsverhältnis der Bindungsaffinität für die MVIIA-Bindungsstelle zur Bindungsaffinität für eine "Stelle-2"-Omega-Conopeptid-Bindungsstelle bewiesen wird, das innerhalb des Bereichs der Selektivitätsverhältnisse liegt, die für die Omega-Conopeptide MVIIA/SNX-111, SNX-199, SNX-236, SNX-239 und TVIA/SNX-185 bestimmt wurden.

6. Verwendung nach einem der Ansprüche 1 bis 5, wobei das Medikament zur Hemmung des Zunehmens neuropathischer Schmerzen an einer verletzten Nervenstelle verwendet wird und perineural an der Stelle der Nervenverletzung verabreicht wird.

7. Stabile Omega-Conopeptid-Formulierung, die ein Omega-Conopeptid und eine Antioxidans-Zusammensetzung umfasst, die die Methioninoxidation wirksam verhindert.

8. Formulierung nach Anspruch 7, worin die Antioxidans-Zusammensetzung einen Carbonsäurepuffer einschließt.

9. Formulierung nach Anspruch 8, worin der Carbonsäurepuffer ein Lactatpuffer ist.

10. Formulierung nach Anspruch 7, worin das Antioxidans Methionin ist.

11. Formulierung nach Anspruch 7, worin die Antioxidans-Zusammensetzung Lactat und Methionin einschließt.

12. Omega-Conopeptid SNX-273 mit der Sequenz:

CKGKGAKCSRLAYDCCTGSCRSGKC.

13. Omega-Conopeptid SNX-279 mit der Sequenz:
CKGKGAKCSRLM(0)YDCCTGSCRSGKC, worin M(O) einen oxidierten Methioninrest anzeigt.

14. Verwendung eines spannungsempfindlichen, Calciumkanal- blockierenden Omega-Conopeptids vom N-Typ zur Herstellung eines Medikaments zur Erzeugung von Analgesie in einem Individuum durch epidurale Verabreichung an das Individuum, umfassend ein Omega-Conopeptid, das

   (a) die elektrisch stimulierte Kontraktion des Meerschweinchenileums wirksam hemmt, und

21

(b) selektiv an die Omega-Conopeptid-MVIIA-Bindungsstellen bindet, die im neuronalen Gewebe vorhanden sind, wobei die Aktivitäten des Omega-Conopeptids bei der Hemmung des Meerschweinchenileums und der Bindung an die MVIIA-Bindungsstelle innerhalb der Bereiche solcher Aktivitäten der Omega-Conotoxine MVIIA und TVIA liegen, und wobei die epidurale Verabreichung über einen spinalen epiduralen Weg über einen Zeitraum erfolgt, dass das Conopeptid in verlängertem Kontakt mit der epiduralen Region ist, bei einer Dosis, die im Bereich der wirksamen intrathekalen analgetischen Dosis ist, die über einen equivalenten Zeitraum verabreicht wird.

15. Verwendung nach Anspruch 14, wobei die epidurale Verabreichung über eine kontinuierliche Infusion erfolgt.

16. Verwendung nach Anspruch 14 oder 15, wobei der verlängerte Kontakt durch Verabreichen des Conopeptids in einer Formulierung zur anhaltenden Freisetzung bewirkt wird.

17. Verwendung nach einem der Ansprüche 14 bis 16, wobei das Conopeptid ausgewählt ist aus der Gruppe, bestehend aus: MVIIA/SNX-111, TVIA/SNX-185, SNX-236, SNX-159, SNX-239, SNX-199, SNX-273, SNX-279 und Derivate davon.

18. Verwendung nach einem der Ansprüche 14 bis 17, wobei die Aktivität, selektiv an die Omega-Conopeptid-MVIIA-Bindungsstellen zu binden, ferner durch das Selektivitätsverhältnis der Bindung an die MVII-Bindungsstelle zur Bindung an eine "Stelle 2"-Omega-Conopeptid-Bindungsstelle bewiesen wird, das innerhalb des Bereichs der Selektivitätsverhältnisse liegt, die für die Omega-Conopeptide MVIIA/SNX-111, SNX-199, SNX-236, SNX-239 und TVIA/SNX-185 bestimmt wurden.

EP 0 835 126 B1

|  | 1 | | | | 5 | | | | | 10 | | | | | 15 | | | | | 20 | | | | | 25 | | | | | 30 |
|---|---|---|---|---|---|---|---|---|---|---|---|---|---|---|---|---|---|---|---|---|---|---|---|---|---|---|---|---|---|---|

**MVIIA/SNX-111**

C K G K G A K C S R L M Y D C C T G S C - R - S G K - C

**MVIIB/SNX-159**

C K G K G A S C H R T S Y D C C T G S C N R - - G K - C

**GVIA/SNX-124**

C K S X G S S C S X T S Y N C C R - S C N X Y T - K R C - - Y

**GVIIB/SNX-178**

C K S X G T X C S R G M R D C C T - S C L L Y S N K - C R R Y

**RVIA/SNX-182**

C K P X G S X C R V S S Y N C C S - S C K S Y - N K K C G

## Fig. 1

EP 0 835 126 B1

```
                1       5        10       15        20       25        30

SVIA/
SNX-    C R S S G S X C G V T S I - C C - G R C - - Y R G K - C T
157


TVIA/
SNX-    C L S X G S S C S X T S Y N C C R - S C N X Y S - R K C R
185


SVIB/
SNX-    C K L K G Q S C R K T S Y D C C S G S C G R - S G K - C
183


MVIIC/
SNX-    C K G K G A P C R K T M Y D C C S G S C G R - R G K - C
230


SNX-    C K G K G A X C R K T M Y D C C S G S C G R - R G K - C
231
```

## Fig. 2

```
              1        5         10        15        20        25

MVIIA     C K G K G A K C S R L M Y D C C T G S C R S G K C-NH₂
(SNX-111)

SNX-190   - - - A - - - - - - - - - - - - - - - A - - - - -  NH₂

SNX-191   - A - - - - - - - - - - - - - - - - - - - - - - -  NH₂

SNX-193   - - - - - - - - - - - - - - - - - - - - - - - - -  G-OH

SNX-194   - - - - - - - - - - - -Nle- - - - - - - - - - - -  NH₂

SNX-195   - - - - - - - - - - - - - - - - - - - - - - A -  NH₂

SNX-196 N - - - - - - - - - - - - - - - - - - - - - - - - -  G-OH

SNX-197NS - - - - - - - - - - - - - - - - - - - - - - - - -  NH₂

SNX-198   - - - - - - - - - - - - - - - - - - - - A - - - -  NH₂

SNX-199   - - - - - - - - - A - - - - - - - - - - - - - - -  NH₂
```

# Fig. 3

EP 0 835 126 B1

|   | 1 |   |   |   | 5 |   |   |   |   | 10 |   |   |   |   | 15 |   |   |   |   | 20 |   |   |   |   | 25 |   |
|---|---|---|---|---|---|---|---|---|---|---|---|---|---|---|---|---|---|---|---|---|---|---|---|---|---|---|
| SNX-200 | - | - | - | - | - | - | A | - | - | - | - | - | - | - | - | - | - | - | - | - | - | - | - | - | - | NH$_2$ |
| SNX-201 | - | - | - | - | - | - | - | R | K | T | S | - | - | - | - | - | - | - | - | - | - | - | - | - | - | NH$_2$ |
| SNX-239 | - | - | - | - | - | - | - | - | L | - | - | - | - | - | - | - | - | - | - | - | - | - | - | - | - | NH$_2$ |
| SNX-240 | Ac- | - | - | - | - | - | - | - | L | - | - | - | - | - | - | - | - | - | - | - | - |   |   |   |   |   |
| SNX-273 | - | - | - | - | - | - | - | - | - | A | - | - | - | - | - | - | - | - | - | - | - | - | - | - | - | NH$_2$ |
| SNX-279 | - | - | - | - | - | - | - | - | - | -M(O)- | - | - | - | - | - | - | - | - | - | - | - | - | - | NH$_2$ |

## Fig. 4

SVIB
(SNX-183) C K L K G Q S C R K T S Y D C C S G S C G R S G K C NH$_2$

SNX-202 - - - - - - - - S R L M - - - - - - - - - - - - NH$_2$

TVIA
(SNX-185) C L S X G S S C S X T S Y N C C R S C N X Y S R K C R NH$_2$

SNX-207 - - - - - - - - R L M - - - - - - - - - - - - - NH$_2$

SNX-236 - - - - - - - - R L M - - - - - - - P - - - - - NH$_2$

## Fig. 5

EP 0 835 126 B1

```
              1         5          10         15          20          25          30

I.
MVIIA   C K G K G A K C S R L M Y D C C T G S C - R - S G K - C

SNX-    C K G K G A K C S L L M Y D C C T G S C - R - S G K - C
239

SNX-    C K G K G A K C S A L M Y D C C T G S C - R - S G K - C
199

MVIIB   C K G K G A S C H R T S Y D C C T G S C N R - - G K - C


II.
TVIA    C L S X G S S C S X T S Y N C C R - S C N X Y S R K - C R

SNX-    C L S X G S S C S R L M Y N C C R - S C N X Y S R K - C R
207

SNX-    C L S X G S S C S R L M Y N C C R - S C N P Y S R K - C R
236


III.
RVIA    C K P X G S X C R V S S Y N C C S - S C K S Y - N K K C G

SVIA    C R S S G S X C G V T S I - C C - G R C - - Y R G K - C T

GVIIA   C K S X G T X C S R G M R D C C T - S C L L Y S N K - C R R Y D

SVIB    C K L K G Q S C R K T S Y D C C S G S C G R - S G K - C

MVIIC   C K G K G A P C R K T M Y D C C S G S C G R - R G K - C

SNX-    C K G K G A X C R K T M Y D C C S G S C G R - R G K - C
231
```

## Fig. 6

Fig. 7

Fig. 8

Fig. 9

Fig. 10

Fig. 11

Fig. 12

**Fig. 13**

**Fig. 14**